# EUROPEAN PATENT SPECIFICATION

(11) **EP 2 329 767 B1**
(45) Date of publication and mention of the grant of the patent: **23.10.2013**
(21) Application number: 08810201.7
(22) Date of filing: 02.09.2008
(51) Int. Cl.: A61B 5/05, A61B 5/117, G01B 7/00, G01N 27/00, G01V 3/08, G01R 29/12

(54) **DETECTOR, DETECTION METHOD, VEIN SENSOR, SCANNED PROBE MICROSCOPE, STRAIN DETECTOR, AND METAL DETECTOR**
DETEKTOR, NACHWEISVERFAHREN, VENENSENSOR, RASTERSONDENMIKROSKOP, BELASTUNGSDETEKTOR UND METALLDETEKTOR
DETECTEUR, PROCEDE DE DETECTION, DETECTEUR DE VEINE, MICROSCOPE A SONDE A BALAYAGE, DETECTEUR DE CONTRAINTE ET DETECTEUR DE METAL

(43) Date of publication of application: 08.06.2011
(73) Proprietor: QFactor Inc., Tokyo 104-0061 (JP)
(72) Inventor: TAKIGUCHI, Kiyoaki, Tokyo (JP); WADA, Takayuki, Tokyo 108-0075 (JP); KOYANO, Hidenori, Tokyo 108-0075 (JP)
(74) Representative: McGowan, Cathrine
(86) International application number: PCT/JP2008/066146
(87) International publication number: WO 2010/026658

(56) References cited:
- EP-A1- 1 658 809
- JP-A- 2003 331 271
- JP-A- 2005 073 974
- JP-A- 2007 282 789
- JP-A- 2008 212 525
- US-A- 5 315 232
- US-A1- 2003 151 240
- US-A1- 2008 060 497

## Description

### Technical Field

The present invention relates to a detection device, detection method, vein sensing device, scanning probe microscope, distortion detection device and metal detection device that are based on a novel principle.

### Background Art

There is a conventional measurement device, proposed by the inventors, which non-invasively measures blood vessels and the like in a living body (see Patent Document 1). The measurement device emits from an emission electrode a quasi-static electric field that enables higher intensity to be obtained than an induction field at each distance corresponding to each of a plurality of frequencies and detects a change in the intensity of the quasi-static electric field of a frequency corresponding to the distance by means of a detection electrode to measure the state of blood vessels and the like.

Meanwhile, there has been a proposed proximity switch equipped with a magnetic field sensitive sensor (see Patent Document 2, for example). In the proximity switch, a magnetic flux-free area is formed by three poles with the same name between U-shaped leg members of a U-shaped permanent magnet having the vertical direction of magnetization. In the area, a sensor is attached that is sensitive to a magnetic field. Above the U-shaped leg members, a planar ferromagnetic trigger member that can come nearer is also provided on a plane parallel to a U-shaped base. As the trigger member comes closer to two poles of the U-shaped leg members, triggering takes place thanks to the offsetting of the magnetic flux-free area, enabling a switching signal of the sensor to be evaluated. FIGS. 49A and 49B show the proximity switch (The diagrams are equivalent to FIG. 1 of Patent Document 2). The principle of the proximity switch is as follows. As shown in FIG. 49A, in the proximity switch, an magnetic flux-free area, or magnetic field-free area, is produced by three north poles between the U-shaped leg members of the U-shaped permanent magnet to eliminate a magnetic field. As shown in 49B, when a ferromagnetic material comes closer as the trigger member, a magnetic field emerges where there is a magnetic field-free area. The magnetic field sensitive sensor attached to the magnetic field-free area detects the change of the magnetic field and predicts that the ferromagnetic material is approaching.

Moreover, it is known that a magnetic field sensitive sensor is disposed in a magnetic field-free area of a unique permanent magnet device (see Patent Document 3).

According to the techniques disclosed in Patent Documents 2 and 3, it is possible to detect an approaching ferromagnetic material that a conventional magnetic field sensitive sensor cannot detect. The principle is that the magnetic field sensitive sensor detects the change of the magnetic field-free area caused by the distortion of the magnetic field associated with the interaction between the magnetic field of the permanent magnet and the ferromagnetic material. However, nothing is disclosed or suggested suggested by Patent Documents 2 and 3 about a detection device that uses an electrode that generates electric charges and an electric field detection element that detects an electric field.

It is know that each tissue of a living body is a dielectric material that has a different electric characteristic (permittivity and conductivity) as well as a different frequency characteristic (see Non-patent Documents 1, 2 and 3). With the use of the characteristics (the difference in electric characteristic between tissues), Impedance CT (Computed Tomography), a method of electrically detecting tissues, has been studied.
Patent Document 1: Japanese Laid-open Patent Publication No. 2005-73974
Patent Document 2: Japanese Laid-open Patent Publication No. 9-511357
Patent Document 3: German Patent Application Publication No. 3901678
Non-patent Document 1: C Gabriel, S Gabriel and E Corthout: "The dielectric properties of biological tissues: I. Literature survey", Phys. Med. Biol. 41(1996)2231-2249
Non-patent Document 2: S Gabriel, R W Lau and C Gabriel: "The dielectric properties of biological tissues: II, Measurements in the frequency range 10Hz to 20GHz", Phys. Med. Biol. 41(1996)2251-2269
Non-patent Document 3: S Gabriel, R W Lau and C Gabriel: "The dielectric properties of biological tissues: III. Parametric models for the dielectric spectrum of tissues", Phys. Med. Biol. 41(1996)2271-2293

Also US 2008/060497 describes driver distraction in a motor vehicle which is assessed by capacitively detecting the driver' s head pose relative to the forward direction of vehicle motion. A symmetrical array of sensor electrodes is disposed in the cockpit ceiling above the driver's head, and pairs of electrodes disposed along varying axes of rotation with respect to the forward direction are successively activated for capacitance measurement. The capacitance measurements are combined to form a signal whose strength depends on the degree of alignment between the driver's head (i.e., the head pose) and the respective axes of rotation, and the driver's head pose is calculated to assess driver distraction.

According to the knowledge of the inventors, for example, even a tiny fatty tissue between a blood vessel and the outer layer of a skin makes it impossible for the measurement device proposed in Patent Document 1 to obtain sensitivity. Thus, it is difficult to accurately measure the state of the blood vessel.

Therefore, the problem to be solved by the invention is to provide a detection device and a detection method that are based on a novel principle and able to detect blood vessels and other various targets with high sensitivity and accuracy.

Another problem to be solved by the invention is to provide a high-performance vein sensing device, scanning probe microscope, distortion detection device and metal detection device that use the above novel detection device.

### Summary of the Invention

Aspects and embodiments of the invention are defined in the appended claims.

### Disclosure of the Invention

After conducting a intense study to solve the above problems, the inventors have found the following technique to solve the above problems and come up with the invention: the technique of generating at least around one straight line m rotationally symmetric electric charges (m is an even number that is greater than or equal to 4) whose total amount of charge is substantially 0 and detecting an electric field on the straight line. The detection device and detection method based on the novel principle cannot be easily invented based on Patent Documents 2 and 3. The following describes the reasons.

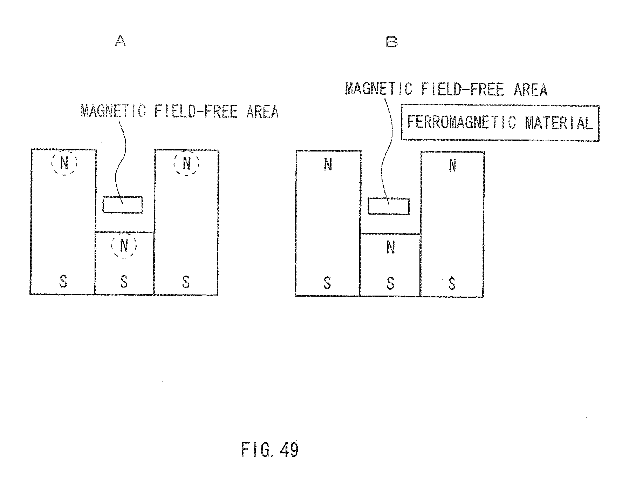

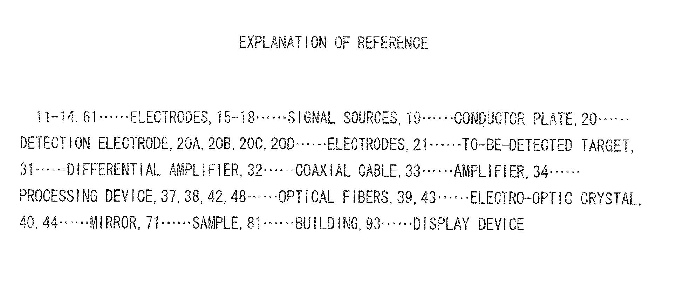

The magnetic field and the electric field are different in that, in the magnetic field, there is no monopole, which corresponds to an electric charge in the electric field. However, both are equivalent to each other if a dipole, instead of a monopole, is used for formation. Accordingly, it seems possible to realize an electric field sensor based on a similar principle to the techniques disclosed in Patent Documents 2 and 3 by replacing the magnetic fields of the two conventional techniques disclosed in Patent Documents 2 and 3 with electric fields as well as magnetic dipoles with electric dipoles. However, in reality, the above is impossible for the following reasons. That is, as a result of the above replacement in the conventional technique, a wire used to obtain signals from an electric field sensitive sensor becomes an electrode, which has impact on the pole structure, leading to a loss of the symmetry. Therefore, an area where the electric field is zero is not formed; the electric field sensor is not realized. In other words, the structure of the magnetic field sensor and the structure of the electric field sensor are not compatible in terms of structure since the physical effects are different. When the above replacement is performed, three N-poles encircled by dotted lines in FIG. 49A correspond to a triple-pole of electric charges. However, the present invention is not realized with the triple-pole; at least a quadruple or higher pole is necessary. Even in this respect, the structure of the magnetic field sensor and the structure of the electric field sensor are not compatible.

That is, to solve the above problems, according to a first aspect of the present invention, a detection device is characterized by comprising:
m electrodes that generate at least around one straight line m rotationally symmetric electric charges (m is an even number that is greater than or equal to 4) whose total amount of charge is substantially 0; and
at least one electric field detection element that detects an electric field on the straight line.

In the detection device, in an exemplary case, the signs of adjacent electric charges of the m electric charges are different (one is positively charged, and the other negatively charged) ; the absolute values of the m electric charges are equal to each other.

In the detection device, on the straight line that acts as a rotational axis of the m rotationally symmetric electric charges, the electric field E₀ is 0 [V/m] due to the overlapping of the electric fields caused by the electric charges. In this case, an area where the electric field E₀=0 [V/m] (point, line, surface) is referred as a unique area. When an area where E₀≈0 [V/m] exists close to the area where E₀=0 [V/m], the area where E₀=0 [V/m] and the area where E₀ is approximately equal to 0 [V/m] are collectively referred to as a unique area. To detect an electric field on a straight line means to detect an electric field of the unique area. In order to improve the sensitivity and accuracy of the detection, it is desirable to bring about a steep change in intensity of the electric field inside and outside the unique area. That is, when the change of the intensity of the electric field is directly measured, it is difficult to detect the change because the amount of change ΔE is small compared with E₀ when the electric field E₀ changes to E₀+ΔE particularly if E₀>>ΔE. Even if the change is amplified by an amplifier, it remains difficult to detect the change because the ratio of ΔE to E₀ remains unchanged. If the electric field is detected in the unique area, it is possible to detect the change of the electric field with high accuracy and sensitivity because the detected intensity of the electric field is equivalent to the amount of change ΔE.

It is desirable that an electric field detection element and a wire extending outside from the electric field detection element be disposed on the straight line. The above means that the wire extending outside the electric field detection element is disposed on an equipotential surface. That is, since the electric field detection element and the wire extending outside from the electric field detection element are disposed on the equipotential surface, the rotational symmetry of m electric charges can be maintained.

In the detection device, by detecting that an electric field of the unique area changes in a way that does not satisfy E₀=0 [V/m] or E₀≈0 [V/m] for some reason, it is possible to detect the breaking of the rotational symmetry of the m electric charges.

The m electric charges are designed to make up, for example, a multipole or planar 2n-pole (n is an integer that is greater than or equal to 2) or to emerge at a vertex of a regular polyhedron or quasi-regular polyhedron. The multipole can be regarded as k dipoles (k is an integer greater than or equal to 2) that are disposed in such a way that the signs of electric charges of the adjacent dipoles are opposite to each other. For example, a quadrupole, octupole or the like can be cited as examples. The planar 2n-pole can be regarded as n dipoles arranged on a plane in such a way that the signs of electric charges of the adjacent dipoles are opposite to each other, for example, including a quadrupole, hexapole and octupole. The regular polyhedron may be a cube, which is used to generate an octupole. A quasi-regular polyhedron may be a truncated octahedron (The Truncated Octahedron), a rhombitruncated cuboctahedron (The Rhombitruncated cuboctahedron), or the like.

For example, in the quadrupole, the charge distribution has invariable two-fold rotational symmetry with respect to the rotation of 180 degrees around a straight line that is a rotational axis. There is only one straight line acting as a rotational axis. In this case, the straight line, or the straight line and an adjacent area, is a unique area. For the quadrupole, changes of an electric field inside and outside the unique area are steep in particular; the quadrupole is exceptionally excellent in the sensitivity and accuracy of detecting the breaking of the charge's rotational symmetry. Therefore, the quadrupole is highly desirable. In the octupole having a three-dimensional structure, the charge distribution has invariable two-fold rotational symmetry with respect to the rotation of 180 degrees around straight lines that are rotational axes. The straight lines acting as rotational axes are three straight lines that cross each other at right angles. In this case, the straight lines, or the straight lines and an adjacent area, are a unique area.

For example, the detection device can detect: (1) a change of the position of at least one electrode among m electrodes; (2) a change of the amount of charge (voltage) of electric changes that emerge on at least one electrode among the m electrodes; and (3) an electric charge (including a charged conductor) or object (which is made of a conductor or dielectric) outside the m electrodes. That is, in the case of (1), when the position of at least one electrode changes for some reason, the rotational symmetry of the m electric charges breaks. Therefore, the electric field on the straight line, which is the rotational axis of the m electric charges, changes in a way that does not satisfy E₀=0 [V/m] or E₀≈0 [V/m]. In the case of (2), when the amount of charge of electric charges that emerge on at least one electrode changes for some reason, the rotational symmetry of the m electric charges breaks. Therefore, the electric field on the straight line, which is the rotational axis of the m electric charges, changes in a way that does not satisfy E₀=0 [V/m] or E₀≈0 [V/m]. In the case of (3), if there is an electric charge or object (which is made of a conductor or dielectric) outside the m electrodes, the rotational symmetry of the m electric charges breaks due to the effects of the electric charge or object. Therefore, the electric field on the straight line, which is the rotational axis of the m electric charges, changes in a way that does not satisfy E₀=0 [V/m] or E₀≈0 [V/m].

The electric charges of the m electrodes may be those caused by applying an alternating voltage to the above electrodes or be static electric charges. In particular, when m electric charges are generated by applying a sine-wave alternating voltage to the m electrodes, it is desirable that d<<λ/2π, where λ represents the length of the sine wave and d represents the length of a dipole that makes up the m electric charges. Thanks to the condition, in an electric field caused by the m electric charges, a quasi-static electric field becomes dominant.

The shape of the m electrodes can be selected when necessary. In general, the m electrodes are point electrodes or planar electrodes. In terms of detection sensitivity and accuracy, it is preferable that the m electrodes be square planar electrodes. When the m electrodes are regarded as one basic unit, the detection device may use only one basic unit or a matrix array electrode where a plurality of basic units are arranged in one-dimensional or two-dimensional array.

The principle and configuration of how the electric field detection element detects do not matter. However, in general, a detection electrode is used. The election electrode may consist of a single electrode. However, in order to improve detection sensitivity and accuracy, it is desirable that the detection electrode be of a dipole type, for example, with a pair of electrodes positioned close to each other. In this case, the difference in potential between the dipole-type electrodes is measured, and the potential difference is amplified by an amplifier or the like when necessary. Thus, it is possible to detect an electric field. The detection electrode may be made by disposing two dipole-type electrodes in such a way that the electrodes cross each other at right angles. An electro-optic crystal may be used for the electric field detection element: it is possible to detect an electric field because of an electro-optic effect, which refers to a change of refractive index caused by the electric field. In this case, for example, a laser beam having a constant polarization plane is emitted to the electro-optic crystal. Then, the polarization plane of the laser beam that has passed through the electro-optic crystal is detected. Subsequently, the change of the refractive index is measured from the rotation angle of the polarization plane. Then, an electric field is detected from the change of the refractive index. Various electro-optic crystals can be used.

The detection device can be used for various apparatuses, devices, systems, microscopes and the like that are used in detecting electric charges on the basis of the detection of the electric field, detecting a conductor or dielectric, detecting a change of the position of an object, or doing other processes.

According to an embodiment, the detection device is characterized in that
m rotationally symmetric electric charges (m is an even number that is greater than or equal to 4) whose total amount of charge is substantially 0 are generated at least around one straight line, and an electric field on the straight line is detected.

According to a second aspect, a detection method is characterized in that
m rotationally symmetric electric charges (m is an even number that is greater than or equal to 4) whose total amount of charge is substantially 0 are generated at least around one straight line, and an electric field on the straight line is detected.

According to a third aspect, a detection device that detects a to-be-detected target or a change of the state of the to-be-detected target is characterized by comprising:
a plurality of electric field applying means for applying electric fields to the to-be-detected target;
electric field detection means for detecting an electric field of a detection area adjacent to the to-be-detected target; and
processing means for detecting a change of the electric field of the detection area detected by the electric field detection means and detecting the to-be-detected target or a change of the state of the to-be-detected target, wherein
when the to-be-detected target is not adjacent to the detection area or is in a predetermined state, a plurality of the electric field applying means applies electric fields in such a way that the electric fields applied from a plurality of the electric field applying means diminish each other and an electric field adjacent to the detection area and the electric field detection means comes to substantially 0.

Here, in an exemplary case, the number of the electric field applying means is m (m is an even number that is greater than or equal to 4). The detection area means an area where the electric field detection means detects an electric field.

According to the first aspect, the detection device may be a vein sensing device, part of a scanning probe microscope, a distortion detection device or a metal detection device.

In this case, the scanning probe microscopes (SPM) include an atom force microscope (AFM), a scanning tunneling microscope (STM), and the like.

According to the invention having the above configuration, the electric field E₀ is equal to 0 [V/m] or approximately equal to 0 [V/m] on the straight line, which is the rotational axis of the m electric charges and in the adjacent unique area. Therefore, the electric field is maintained when the rotational symmetry of the m electric charges is kept. When the rotational symmetry of the m electric charges breaks for some reason, the electric field of the unique area changes in a way that does not satisfy E₀=0 [V/m] or E₀≈0 [V/m]. Moreover, the electric field detection element and the wire thereof are provided on the straight line, which is a rotational axis. Therefore, the wire does not affect the rotational symmetry of the m electric charges. That is, the rotational symmetry of the m electric charges is maintained. Therefore, the wire does not affect the detection capability. Moreover, it is possible for the detection device and method to obtain sufficiently large sensitivity even when fat tissue exists between the outer layer of a skin and a blood vessel. Therefore, it is possible to detect blood vessels with high sensitivity and accuracy.

According to the present invention, it is possible to realize a detection device that is based on a novel principle and able to detect blood vessels and other various targets with high sensitivity and accuracy. It is also possible to realize a high-performance vein sensing device, scanning probe microscope, distortion detection device and metal detection device that use the above detection device.

### Brief Description of the Drawings

FIG. 1 is a schematic diagram showing a quadrupole according to the present invention.
FIGS. 2A to 2C are schematic diagrams showing electric fields generated by the quadrupole and the distribution of electric potential, according to the present invention.
FIGS. 3A to 3C are schematic diagrams showing enlarged portions of the electric field generated by the quadrupole and an enlarged portion of the distribution of electric potential, according to the present invention.
FIG. 4A is a schematic diagram showing a hexapole and FIG. 4B is a schematic diagram showing an octupole according to the present invention.
FIG. 5 is a schematic diagram showing the distributions of electric fields on lines that pass through unique areas of a quadrupole, hexapole and octupole, according to the present invention.
FIGS. 6A to 6C are schematic diagrams illustrating the effects of the distances between electric charges of a quadrupole, hexapole and octupole, respectively, according to the present invention.
FIG. 7 is a schematic diagram showing the distributions of the electric fields on x-axes shown in FIGS. 6A, 6B and 6C.
FIGS. 8A and 8B are schematic diagrams showing a three-dimensional octupole according to the present invention.
FIGS. 9A to 9C are schematic diagrams illustrating an electric field generated by the three-dimensional octupole and the distribution of electric potential, according to the present invention.
FIGS. 10A to 10C are schematic diagrams showing an enlarged portion of the electric field generated by the three-dimensional octupole and an enlarged portion of the distribution of electric potential, according to the present invention.
FIG. 11 is a schematic diagram showing the arrangement of electric charges with electric charges being disposed at each vertex of a truncated octahedron.
FIGS. 12A and 12B are perspective views showing a detection device according to a first embodiment of the present invention as well as a side view showing the detection device.
FIG. 13 is a side view illustrating an operation of the detection device according to the first embodiment of the present invention.
FIGS. 14A and 14B are two-dimensional views illustrating an operation of the detection device according to the first embodiment of the present invention.
FIG. 15 is a schematic diagram showing a formulated model used for an electromagnetic simulation conducted on the detection device according to the first embodiment of the present invention.
FIG. 16 is a schematic diagram showing the results of an electromagnetic simulation conducted on the detection device according to the first embodiment of the present invention.
FIGS. 17A to 17D are schematic diagrams showing a change in the horizontal position of a to-be-detected target corresponding to each of the results shown in FIG. 16.
FIG. 18 is a schematic diagram showing the results of an electromagnetic simulation conducted on the detection device according to the first embodiment of the present invention.
FIG. 19 is a schematic diagram showing the results of an electromagnetic simulation conducted on the detection device according to the first embodiment of the present invention.
FIGS. 20A to 20C are schematic diagrams showing a change in the size of the to-be-detected target corresponding to each of the results shown in FIG. 19.
FIG. 21 is a schematic diagram showing the results of an electromagnetic simulation conducted on the detection device according to the first embodiment of the present invention.
FIG. 22 is a schematic diagram showing the results of an electromagnetic simulation conducted on the detection device according to the first embodiment of the present invention.
FIG. 23 is a schematic diagram showing the results of an electromagnetic simulation conducted on the detection device according to the first embodiment of the present invention.
FIGS. 24A and 24B are schematic diagrams showing changes in the distribution of electric fields when an electrode of the detection device deviates according to the first embodiment of the present invention.
FIGS. 25A and 25B are schematic diagrams showing the distribution of an electric field on the x-axis shown in FIGS. 24A and 24B.
FIGS. 26A and 26B are schematic diagrams showing changes in the distribution of an electric field when an electric charge of an electrode of the detection device changes according to the first embodiment of the present invention.
FIGS. 27A and 27B are schematic diagrams showing the distribution of an electric field on the x-axis shown in FIGS. 26A and 26B.
FIGS. 28A and 28B are schematic diagrams illustrating a detection system used in the detection device according to the first embodiment of the present invention.
FIGS. 29A and 29B are schematic diagrams illustrating a detection system used in a detection device according to a second embodiment of the present invention.
FIG. 30 is a schematic diagram illustrating the detection system used in the detection device according to the second embodiment of the present invention.
FIG. 31 is a schematic diagram showing a formulated model used in an electromagnetic simulation conducted on a vein sensing device according to a third embodiment of the present invention.
FIG. 32 is a schematic diagram showing the structure of an electrode and a numerical phantom that are used in an electromagnetic simulation conducted on the vein sensing device according to the third embodiment of the present invention.
FIG. 33 is a schematic diagram showing the results of an electromagnetic simulation conducted on the vein sensing device according to the third embodiment of the present invention.
FIG. 34 is a schematic diagram showing the results of an electromagnetic simulation conducted on the vein sensing device according to the third embodiment of the present invention.
FIG. 35 is a schematic diagram showing the results of an electromagnetic simulation conducted on the vein sensing device according to the third embodiment of the present invention.
FIG. 36 is a schematic diagram showing the results of an electromagnetic simulation conducted on the vein sensing device according to the third embodiment of the present invention.
FIG. 37 is a schematic diagram showing the results of an electromagnetic simulation conducted on the vein sensing device according to the third embodiment of the present invention.
FIG. 38 is a schematic diagram showing a vein sensing device according to a fourth embodiment of the present invention.
FIG. 39 is a two-dimensional view showing a portion of a matrix array electrode used in the vein sensing device according to the fourth embodiment of the present invention.
FIG. 40 is a schematic diagram showing the results of an electromagnetic simulation conducted on the vein sensing device according to the fourth embodiment of the present invention.
FIG. 41 is a schematic diagram showing the results of an electromagnetic simulation conducted on the vein sensing device according to the fourth embodiment of the present invention.
FIGS. 42A and 42B are two-dimensional views showing a portion of a matrix array electrode used in the vein sensing device according to the fourth embodiment of the present invention.
FIG. 43 is a scanning probe microscope according to a fifth embodiment of the present invention.
FIG. 44 is a schematic diagram showing a distortion detection device according to a sixth embodiment of the present invention.
FIG. 45 is a schematic diagram showing a metal detection device according to a seventh embodiment of the present invention.
FIG. 46 is a schematic diagram illustrating a detection system used in the metal detection device according to the seventh embodiment of the present invention.
FIG. 47 is a schematic diagram showing a scanning probe microscope according to an eighth embodiment of the present invention.
FIG. 48 is a schematic diagram showing a detection device used as a probe in the scanning probe microscope according to the eighth embodiment of the present invention.
FIG. 49 is a schematic diagram illustrating a technique disclosed in Patent Document 2.

### Best Mode for Carrying Out the Invention

The following describes an embodiment of the present invention with reference to the accompanying drawings.

What is first described is a specific example of the configuration of electrodes used in generating m electric charges in a detection device of the present invention as well as an specific example of a unique area generated by the m electric charges.

### (1) Quadrupole

In order to obtain a quadrupole, four electrodes are disposed at the vertexes of a square. The same voltage is applied to the electrodes on a diagonal of the square. The voltage whose polarity is inverted against the voltage is applied to the electrodes on the other diagonal. The applied voltage is a direct-current or alternating voltage.

When a direct-current voltage is applied, the electrodes on one diagonal are charged with +Q [C], and the electrodes on the other diagonal with -Q [C].

When an alternating current is applied, for example, a sine-wave voltage is applied to electrodes on one diagonal. A sine-wave voltage whose phase has been shifted by 180 degrees from the above sine-wave voltage is applied to the electrodes on the other diagonal.

According to the present invention, to apply a direct-current voltage to the electrodes in principle means to apply an alternating voltage to the electrodes. Therefore, what is described hereinafter is a model where a static electric charge is changed by applying a direct-current voltage to the electrodes.

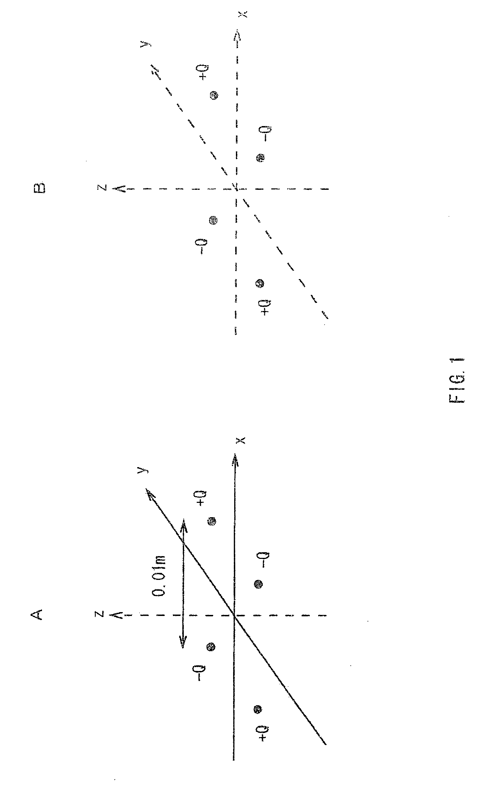

When absolute values of electric charges of all the four electrodes are equal to each other, a unique area emerges exactly at the center of the four electrodes. FIGS. 1A and 1B show the electric charge distribution at the time. In this case, four point charges +Q, -Q, +Q and -Q are disposed on the xy-plane. In FIG. 1A, the z-axis, indicated by dotted line, represents a unique area whose electric field is 0 [V/m]. In FIG. 1B, the x-, y- and z-axes, indicated by dotted lines, represent areas where electric potentials are 0[V].

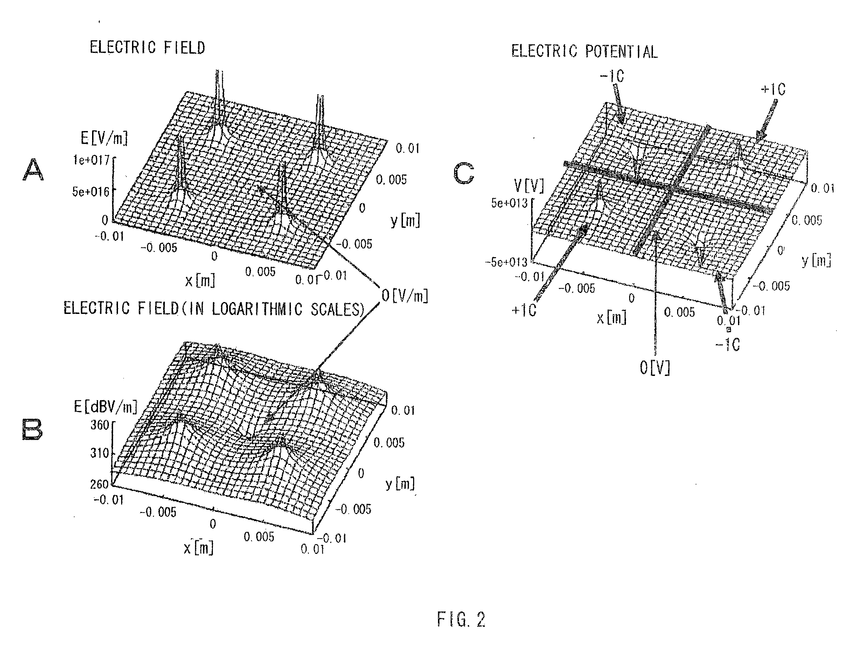

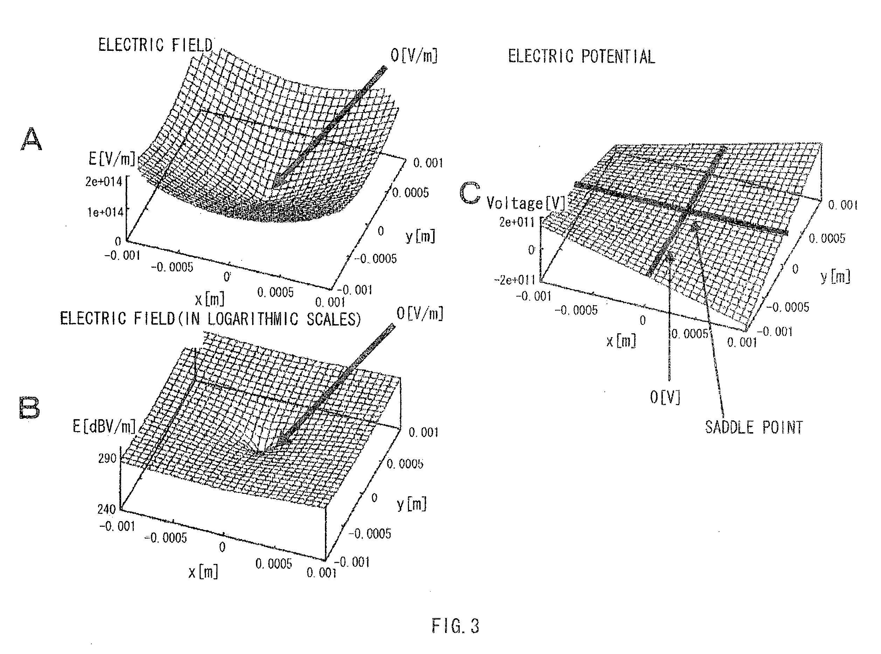

FIGS. 2A and 2B are diagrams drawn after, as for the electric charge distributions shown in FIGS. 1A and 1B, electric fields within the xy-plane are calculated thanks to the superimposed electric fields generated by the four point charges and mapped. FIG. 2A shows the electric fields E [V/m] in logarithmic scales. FIG. 2B shows the electric fields E [V/m] in linear scales. FIGS. 3A and 3B are enlarged views of the central portions of FIGS. 2A and 2B. FIG. 2C shows the electric potential distributions corresponding to the electric field distributions shown in FIGS. 2A and 2B. FIG. 3C is an enlarged view of the central portion of FIG. 2C. However, Q=1[C]; the distance between point charges is 0.01m.

It is clear from FIGS. 2B and 3B that a unique area emerges at the center of the four electrodes (where x=0 and y=0). In FIG. 2C, a black bold line represents an area where electric potential is 0[V].

### (2) Planar 2n-pole

A planar 2n-pole is made by generalizing the above quadrupole on a plane. The planar 2n-pole is a structure where electric charges are disposed at the positions corresponding to the vertexes of a shape having equal sides and 2n corners (a square, an equilateral hexagon, an equilateral octagon or the like) so that the adjacent electrodes have opposite polarities. In this case, the central portion of the shape having equal sides and 2n corners turn out to be a unique area.

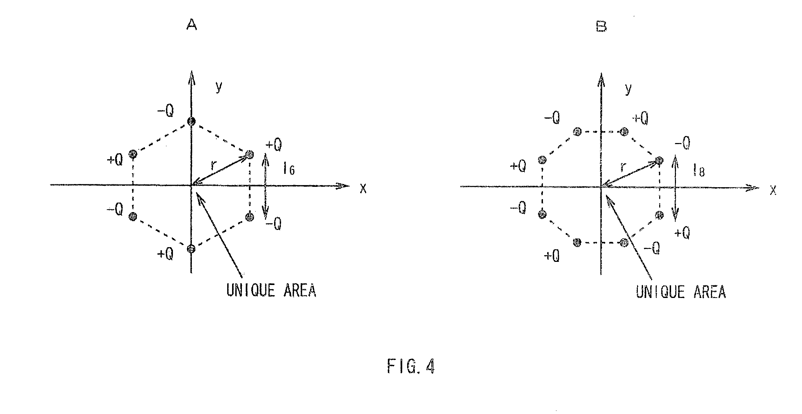

FIG. 4A shows the case where a hexapole (when n=3) is used. FIG. 4B shows the case where an octupole (when n=4) is used.

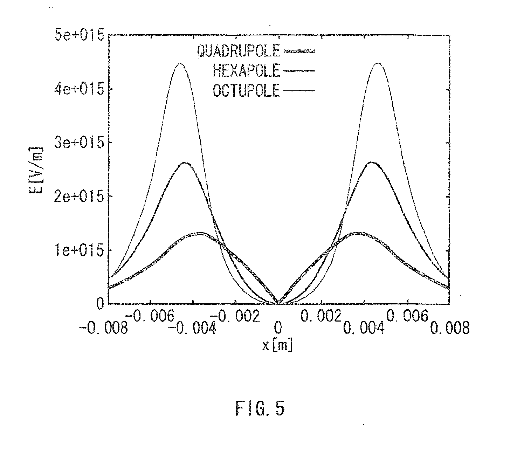

The sharpness of the unique areas at the central portions of the quadrupole, the hexapole and the octupole is evaluated. FIG. 5 shows the results thereof. It is obvious from FIG. 5 that as for the planar 2n-pole, the quadrupole has the sharpest unique area and it is possible to sensitively detect the breaking of the symmetry.

It is clear from FIG. 5 that as for the planar 2n-pole, the electric fields inside and outside the unique area change more gently as n increases. The reason is as follows.

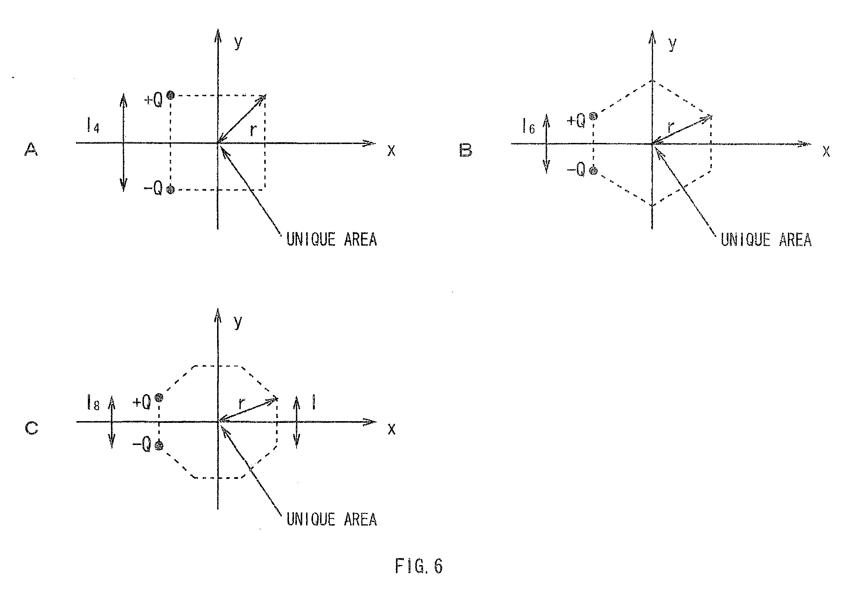

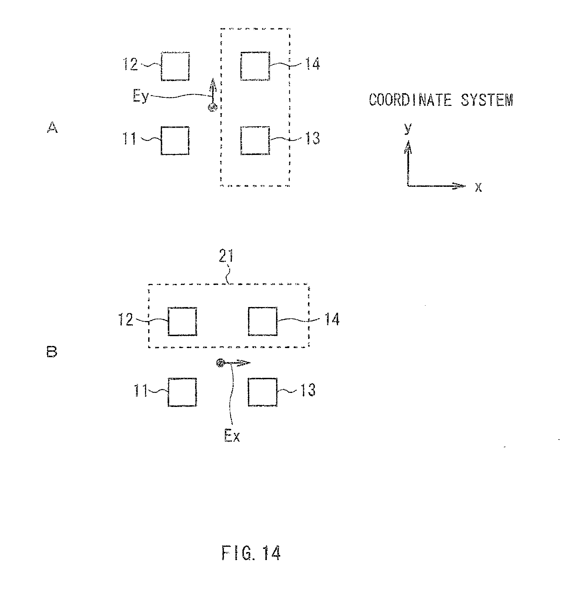

As shown in FIGS. 6A, 6B and 6C, a focus is put on the electric charges (+Q, -Q) on the adjacent two vertexes of a polygon (a square, an equilateral hexagon, and an equilateral octagon). If the center of the polygon remains constant, i.e. the distance r from the unique area to each vertex remains constant, the distance 1 (= the length of a side of the polygon) between the adjacent electric charges becomes smaller as n increases. In the cases of FIGS. 6A, 6B and 6C, 14>16>18.

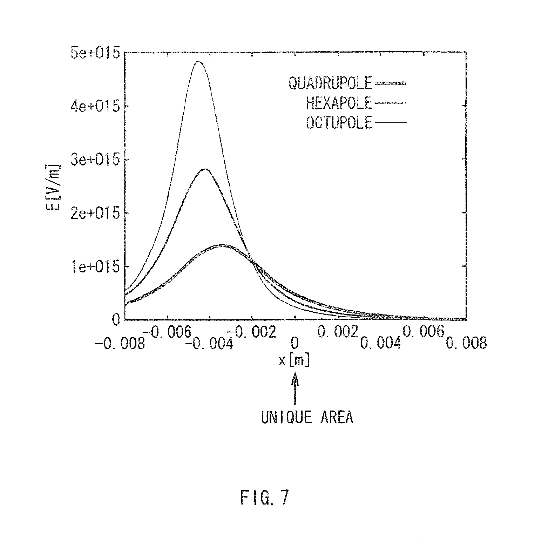

As 1 becomes smaller relative to the distance r from each vertex of the polygon to the unique area, the electric fields caused by two electric charges diminish each other, resulting in the smaller electric field strength of the unique area. That is, as 1 increases, the strength of the electric field of the unique area becomes larger. Specifically, when electric charges are disposed on the vertexes of a square, the strength of the electric field of the unique area grows to a maximum. FIG. 7 is a graph showing the strength of the electric fields on the x-axis shown in FIG. 6A, 6B and 6C. It is obvious from FIG. 7 that the strength of the electric field caused by two electric charges of the quadrupole is the largest compared with the strength of the electric field caused by two electric charges of any other planar 2n-pole.

### (3) Three-dimensional octupole

In order to obtain a three-dimensional octupole, eight electrodes are disposed at the vertexes of a cube.

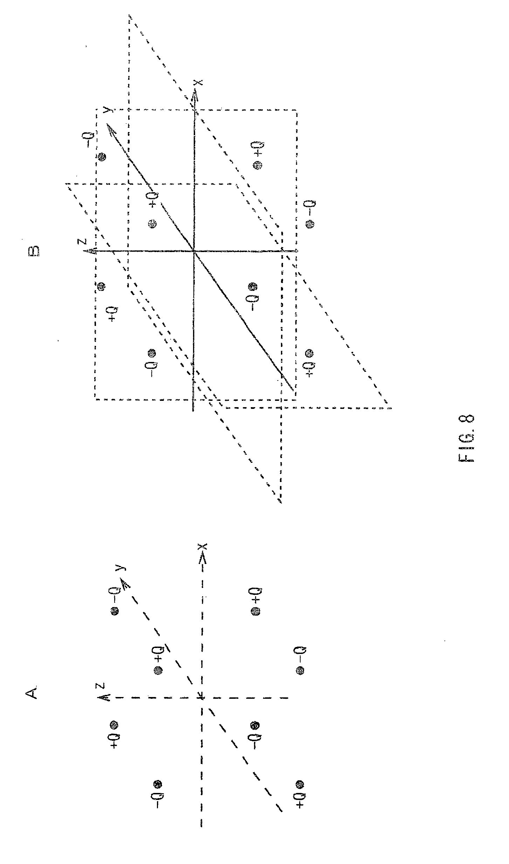

When the absolute values of all the electric charges of the eight electrodes are equal to each other, a unique area emerges right at the center of the eight electrodes, i.e. the center of the cube. FIGS. 8A and 8B show the electric charge distribution at the time. In FIG. 8A, the x-, y- and z-axes, indicated by dotted lines, represent a unique area where an electric field is 0 [V/m]. In FIG. 8B, the three planes (xy-plane, yz-plane, and zx-plane), indicated by dotted lines, represents an area where electric potential is 0[V].

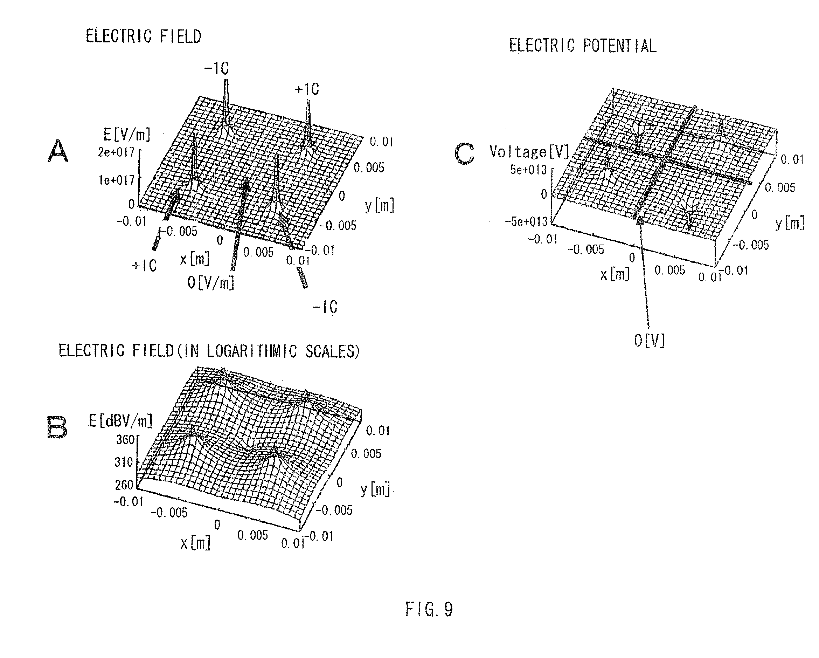

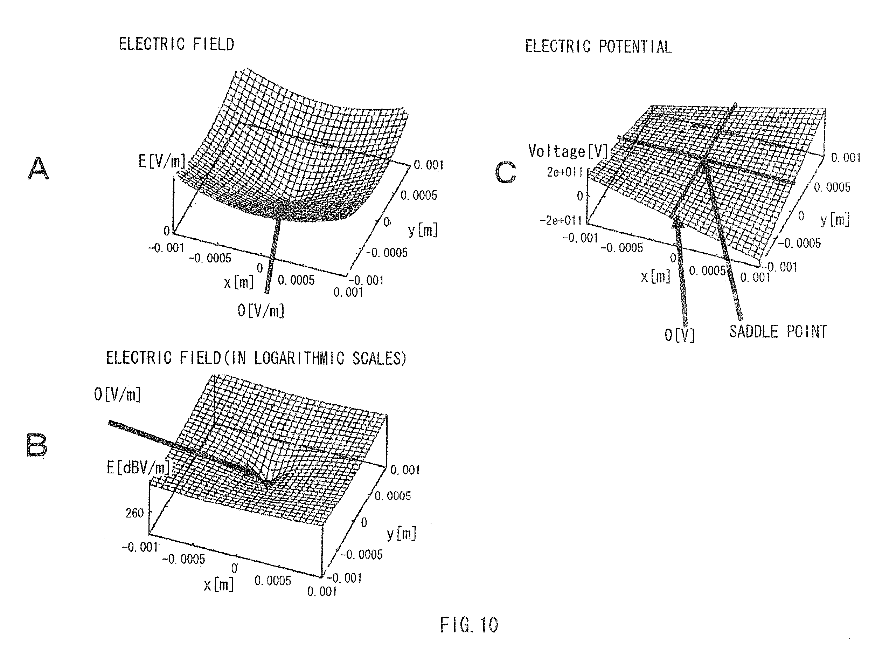

FIGS. 9A and 9B show a diagram generated by calculating the electric field on the underside of the cube in the electric charge distribution shown in FIGS. 8A and 8B and mapping. FIG. 9A shows the electric fields E [V/m] in logarithmic scales. FIG. 9B shows the electric fields E [V/m] in linear scales. FIGS. 10A and 10B are enlarged views of the central portions of FIGS. 9A and 9B. FIG. 9C shows the electric potential distribution corresponding to the electric field distribution shown in FIGS. 9A and 9B. FIG. 10C is an enlarged view of the central portion of FIG. 9C. However, Q=1[C] and the distance between point charges is 0.01m.

It is clear from FIGS. 9B and 10B that a unique area emerges at the center (where x=0, y=0 and z=0) of the eight electrodes. The black bold lines in FIG. 9C represent an area where electric potential is 0[V].

### (4) Examples of other multipole structures

If all the faces of a regular polyhedron or quasi-regular polyhedron are in a shape having 2n corners, a multipole can be made by applying electric charges to the vertexes of each face in such a way that the adjacent polarities are inverted. In this case, a rotationally symmetric figure emerges with a normal line of the center of each face serving as an axis. That is, the multipole is made so that the normal line of the center of each face serves as a unique area. The above configuration is made possible only by a cube among regular polyhedrons. The above configuration corresponds to the above octupole.

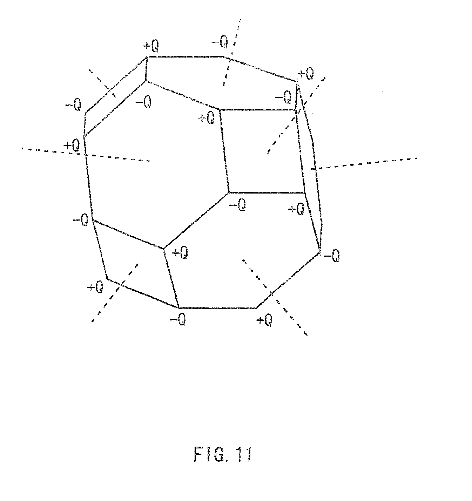

The above configuration is made possible by a truncated octahedron, a rhombitruncated cuboctahedron and the like among quasi-regular polyhedrons. FIG. 11 shows the case where a multipole is made by applying electric charges to the vertexes of each face of the truncated octahedron in such a way that the adjacent polarities are inverted.

Based on the above assumption, embodiments of the present invention will be described.

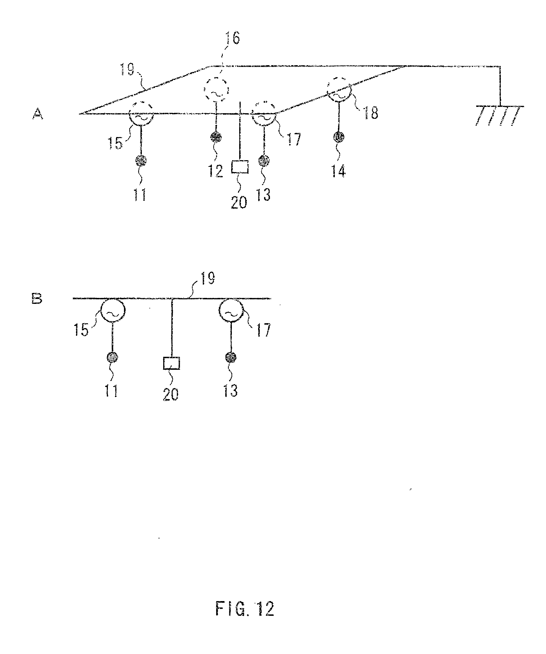

FIGS. 12A and 12B show a detection device according to a first embodiment of the present invention. FIG. 12A is a perspective view. FIG. 12B is a lateral view.

As shown in FIGS. 12A and 12B, in the detection device, four electrodes 11 to 14 are disposed on the vertexes of a square. The electrodes 11 to 14 make up a quadrupole. The electrodes 11 to 14 are connected to a conductor plate 19 through signal sources 15 to 18. The conductor plate 19 is grounded and provides an electric potential reference. The same sine-wave voltage is applied by the signal sources 15 and 18 to the electrodes 11 and 14. What is applied to the electrodes 12 and 13 by the signal sources 16 and 17 is the sine-wave voltage whose phase is shifted by 180 degrees from the sine-wave voltage applied to the electrodes 11 and 14. At this time, a unique area emerges on a vertical line that passes through the center of the four electrodes 11 to 14. A detection electrode 20 is provided in the unique area to detect an electric field. When there is no to-be-detected target, E=0[V/m] or E≈0[V/m] in the electric field detected by the detection electrode 20 or electric field of the unique area because of the symmetry of the electric charges of the electrodes 11 to 14.

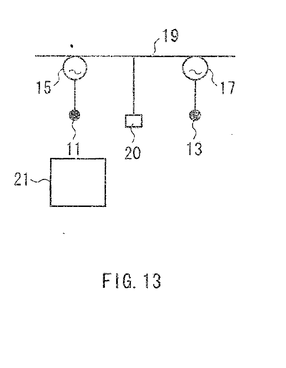

Suppose that a to-be-detected target 21 is beneath the electrodes 11 and 12 as shown in FIG. 13. In this case, the strength of an electric field emanating from the electrodes 13 and 14 is large. Meanwhile, the strength of an electric field emanating from the electrodes 11 and 12 is small. Thanks to the above difference, the electric field detected by the detection electrode 20 changes in a way that does not satisfy E=0 [V/m] or E≈0[V/m]. If the electric field detected by the detection electrode 20 does not satisfy E=0[V/m] or E≈0[V/m], it is possible to detect that the to-be-detected target 21 gets closer to the electrodes 11 to 14.

In this case, in detecting the to-be-detected target 21, the difference in electric characteristic (permittivity and conductivity) between the to-be-detected target 21 and the surrounding space is used. Specifically, for example, the to-be-detected target 21 is a conductor in the atmosphere, a dielectric in the atmosphere (it is easy to detect a dielectric whose permittivity is larger than the permittivity of the atmosphere), the blood in the fat (the blood is higher in electric conductivity than the fat), or the like.

If the to-be-detected target 21 is in the shape of a rod or line (For example, the to-be-detected target 21 is a vein), it is preferable that not only the electric field strength but also an electric field vector to be detected be taken into account. The following explains such a case.

If the rod- or line-shaped to-be-detected target 21 is positioned beneath two out of the four electrodes 11 to 14, the component of the electric field that emerges is in the same direction as that of the to-be-detected target 21 (longitudinal direction). For example, as shown in FIG. 14A, if the rod-shaped to-be-detected target 21 is positioned beneath the electrodes 13 and 14 as shown in FIG. 14A, an electric field component Ey of the y-direction is dominant. If the rod-shaped to-be-detected target 21 is positioned beneath the electrodes 12 and 14 as shown in FIG. 14B, an electric field component Ex of the x-direction is dominant. In this case, therefore, it is preferable to provide the detection electrode 20 able to detect both the electric field component Ex of the x-direction and the electric field component Ey of the y-direction. Therefore, it is possible to detect the direction of the to-be-detected target 21 thanks to the electric field components Ex and Ey and the magnitude of the electric field components Ex and Ey.

In the detection device, the arrangement of the electrodes 11 to 14, the shape of the electrodes 11 to 14, and the distance from the electrodes 11 to 14 to the to-be-detected target 21 correlate with the amount of detection (electric field strength). Accordingly, the following describes the results of formulation based on an electromagnetic simulation for the above.

The conditions of the simulation are as follows.

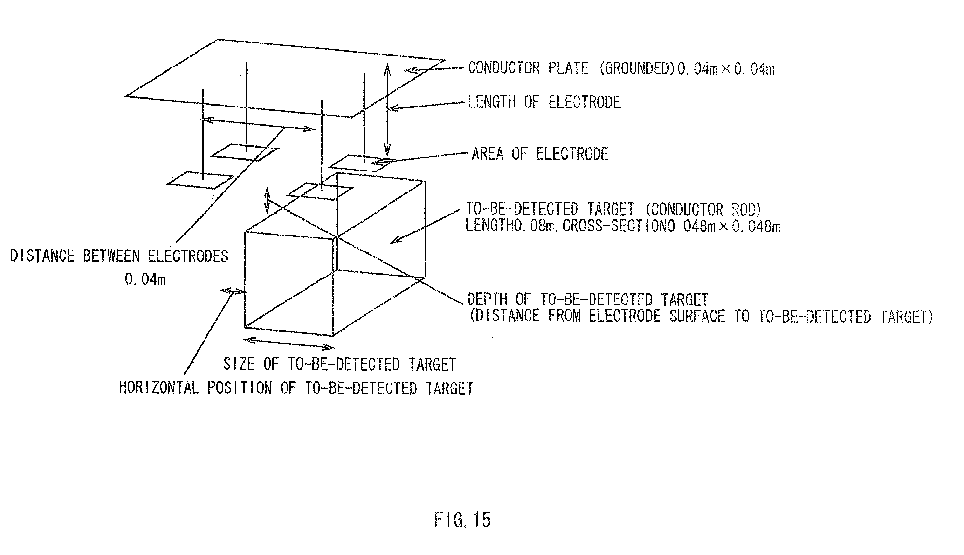

FIG. 15 shows a formulation model. The dimensions of a conductor plate are 0.04m×0.04m. The distance between electrodes is 0.04m. The length of a to-be-detected target is 0.08m. The dimensions of the section of a conductor rod are 0.048m×0.048m.

Simulation software: EEM-FDM by Information and Mathematical Science Laboratory Inc.

Calculation method: Maxwell's equations calculated by the finite-difference frequency-domain

Calculation range: x: -0.04 to 0.04m, y: -0.06 to 0.06m, z: -0.05 to 0.05m
Mesh size: 0.002m
Frequency: 1MHz, Amplitude: 1V

The following shows formulation items
a. The horizontal position of a to-be-detected target - the electric field strength of a unique area
b. The depth of a to-be-detected target - the electric field strength of a unique area
c. The size of a to-be-detected target - the electric field strength of a unique area
d. The distance between electrodes - the depth of detection
e. The length of an electrode - the depth of detection
f. The size of a tip of an electrode - the depth of detection

The above will be described one by one.

### a. The horizontal position of a to-be-detected target - the electric field strength of a unique area

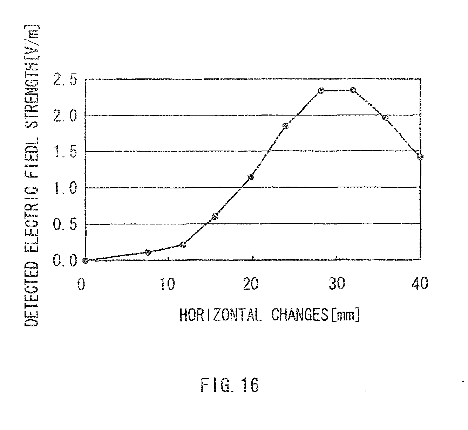

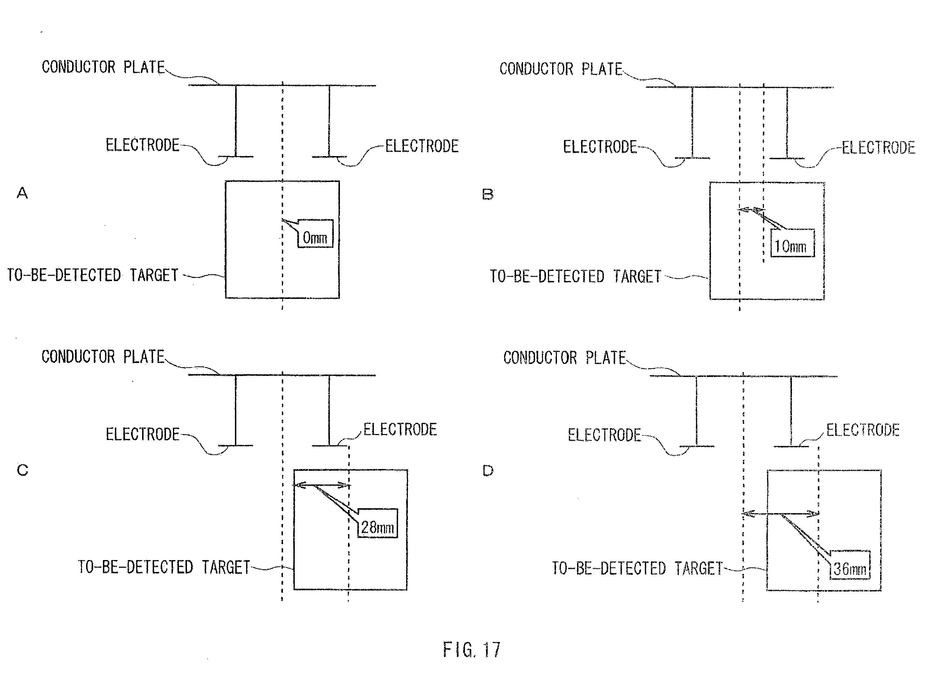

FIG. 16 shows the results of the simulation. It is clear from FIG. 16 that the detected electric field strength is dependent on the relationship between the horizontal position of a to-be-detected target and that of an electrode. FIG. 17 shows the relationship between the horizontal position of the detection device and that of the to-be-detected target. It is clear from FIGS. 16 and 17 that, when the edge of a to-be-detected conductor rod is positioned at the center (unique area) of the electrode, i.e. when the change in the horizontal direction of the horizontal axis of FIG. 16 is about 28mm, the largest electric field strength is detected.

### b. The depth of a to-be-detected target - the electric field strength of a central point

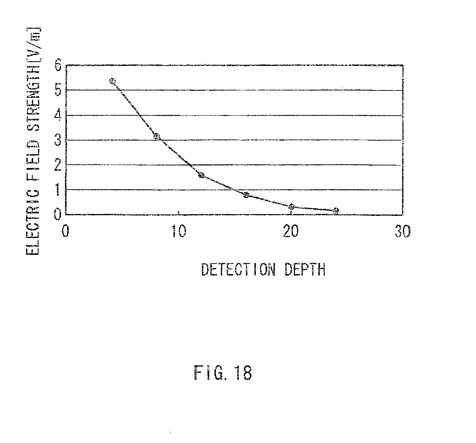

FIG. 18 shows the results of the simulation. It is clear from FIG. 18 that as the to-be-detected target draws closer, i.e. the depth of detection becomes shallower, the electric field strength rises and can be easily detected.

### c. The size of a to-be-detected target - the electric field strength of a unique area

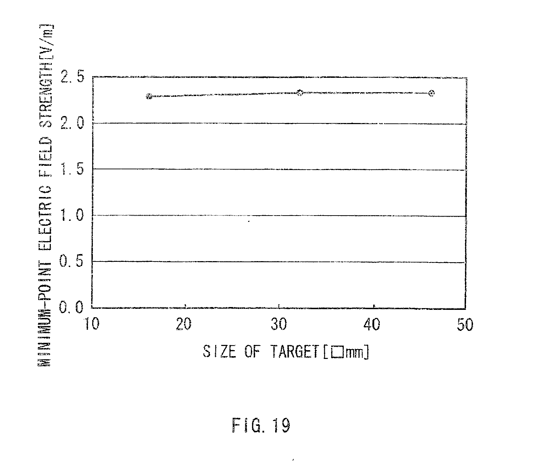

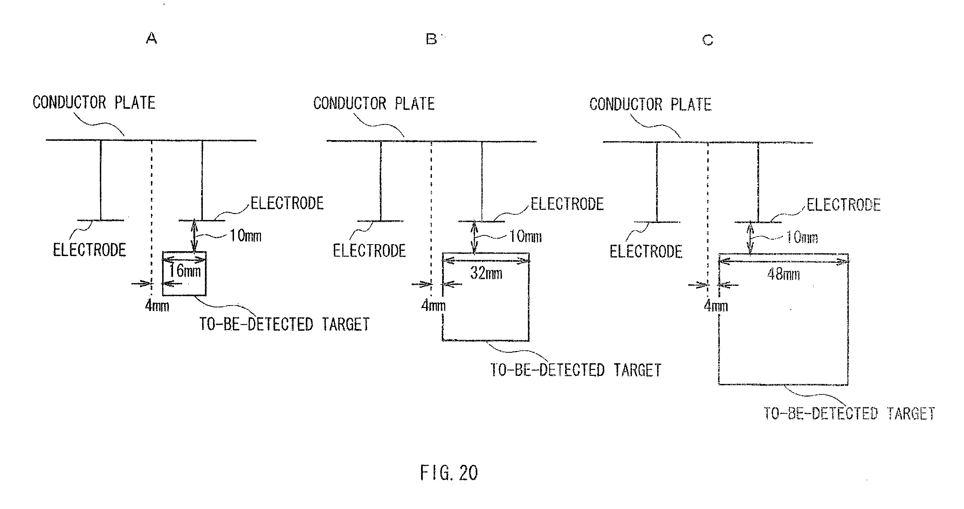

FIG. 19 shows the results of the simulation. FIG. 20 shows a lateral view when the size of the to-be-detected target is changed. It is clear from FIG. 19 that even if the size of the to-be-detected target is changed, the electric field strength of the unique area remains almost unchanged. There is little impact of the size of the to-be-detected target.

### d. The distance between electrodes - the depth of detection

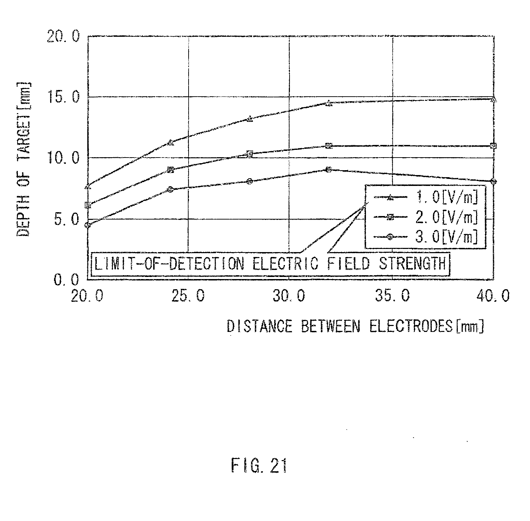

FIG. 21 shows the results of the simulation. It is clear from FIG. 21 that as the distance between electrodes gets larger, it tends to be easier to detect the to-be-detected target positioned at depth.

### e. The length of an electrode - the depth of detection

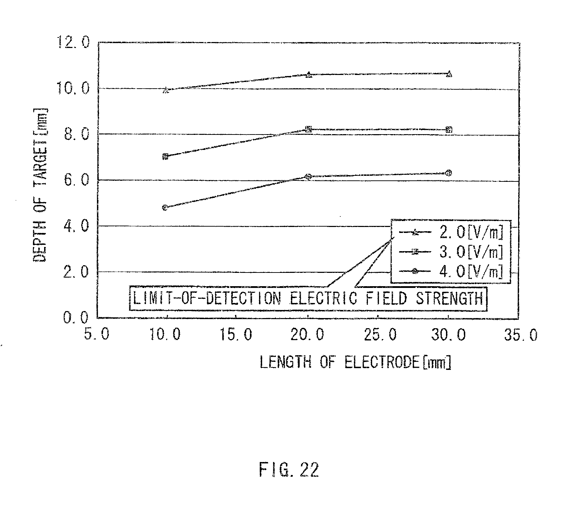

FIG. 22 shows the results of the simulation. It is clear from FIG. 22 that the performance of detecting the to-be-detected target remains almost unchanged if the length of the electrodes is about 20mm or more.

### f. The size of a tip of an electrode - the depth of detection

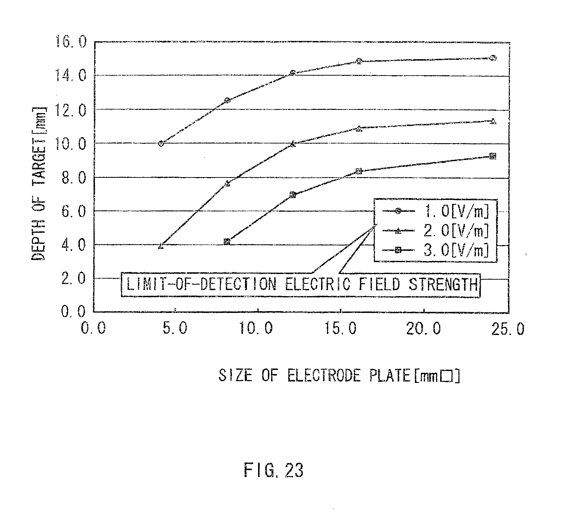

FIG. 23 shows the results of the simulation. It is clear from FIG. 23 that the larger area of a plate of the tip of the electrode is favorable to detecting.

The summary of the above is: the electric field strength of the unique area is dependent on the horizontal position and depth of the to-be-detected target. The sensitivity for changes in the horizontal position is significantly high. The amount of detection becomes smaller for the to-be-detected target positioned at depth. When the electrode is a plate, it is possible to detect the to-be-detected target positioned at depth as the area of the plate gets larger.

The following describes what the detection device detects.

What is described first is the case where changes in the positions of the electrodes 11 to 14 are detected by the detection device.

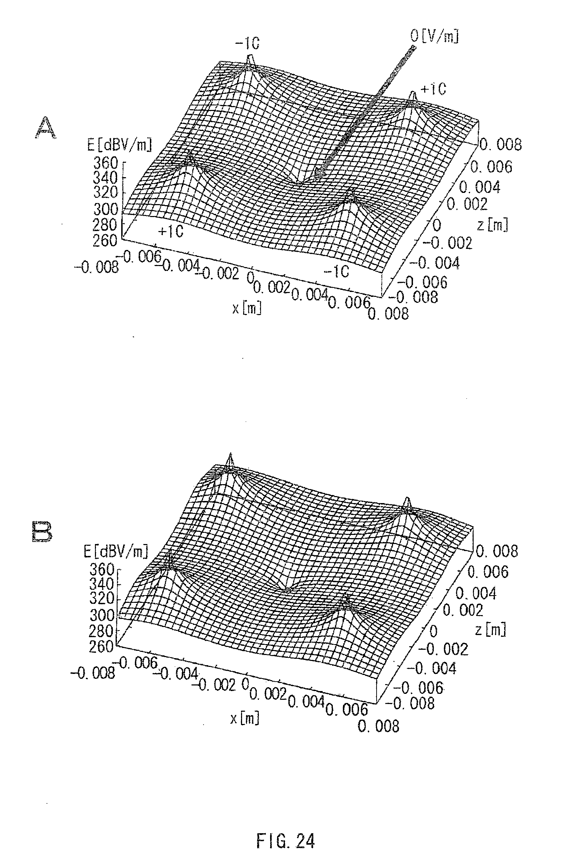

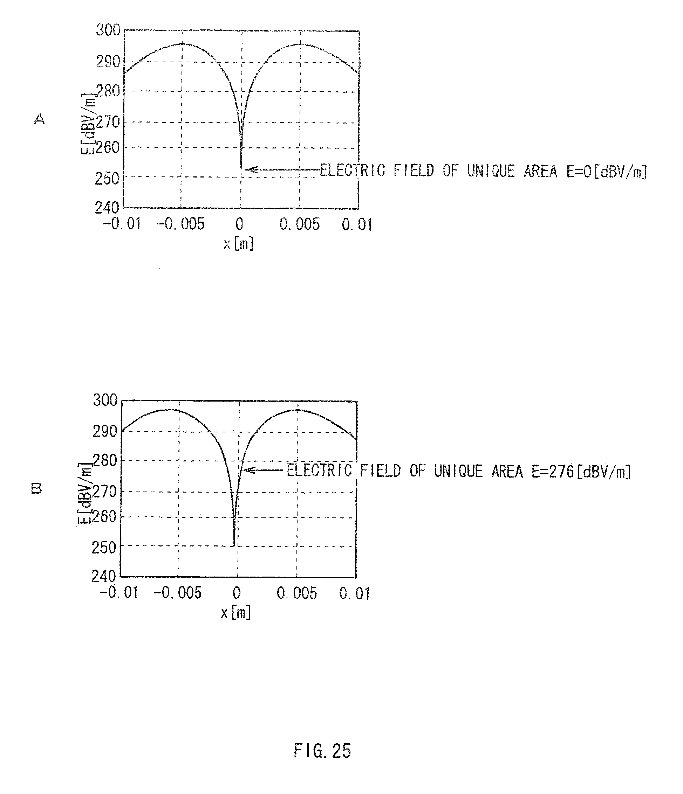

Suppose that the electric charges have moved as the positions of some of the electrodes 11 to 14 have changed. The following looks at the case where the electrodes 11 and 12 have moved by about 0.001 [m] to the left in FIGS. 12A and 12B. FIG. 24A shows the results of mapping the electric field strength of the quadrupole before the positions of the electrodes 11 and 12 change. FIG. 24B shows the results of mapping the electric field strength of the quadrupole after the positions of the electrodes 11 and 12 have changed by about 0.001 [m]. FIG. 25A is a graph showing the electric field strength on the line y=0 in FIG. 24A. FIG. 25B is a graph showing the electric field strength on the line y=0 in FIG. 24B. The horizontal axis represents the x-direction position.

It is clear from FIGS. 24A and 24B as well as FIGS. 25A and 25B that the electric field at a position where the electric field strength is 0 is:
E=0[V/m] with x=0 [m] in the case where the positions of the electrodes 11 and 12 have not changed; or
E=36.1[V/m] with x=0[m] in the case where the positions of the electrodes 11 and 12 have changed to the left.

In this case, a portion of the unique area where the electric field strength equals 0 moves to the left (the negative direction of x) by 0.001/2=0.0005[m]. However, since the change of the electric field around the unique area is steep, the amount of detection of the electric field is large. In general, when two of the electrodes move only by a in the direction parallel to that of the x-axis, the unique area moves by a/2. It is clear from the graphs of FIGS. 25A and 25B that the change of the electric field in the unique area is large.

The following describes the case where the detection device detects changes in the amounts of charges of the electrodes 11 to 14.

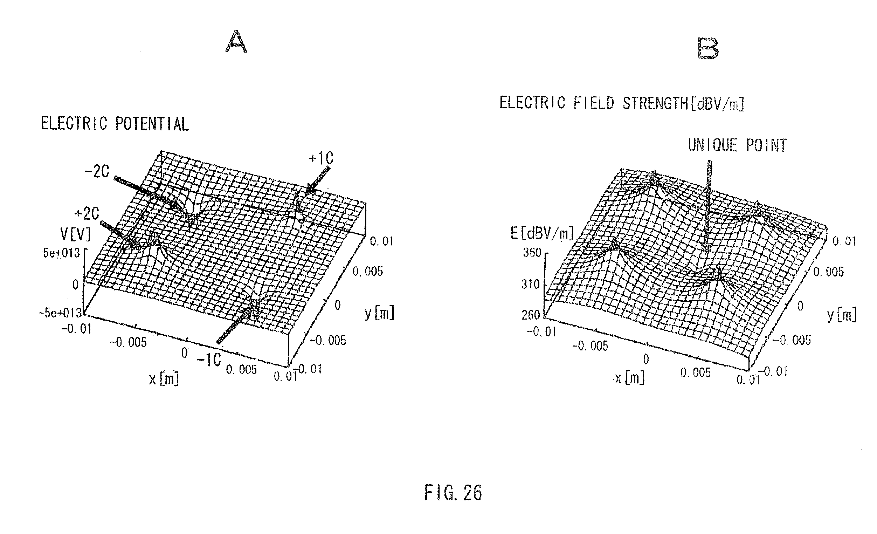

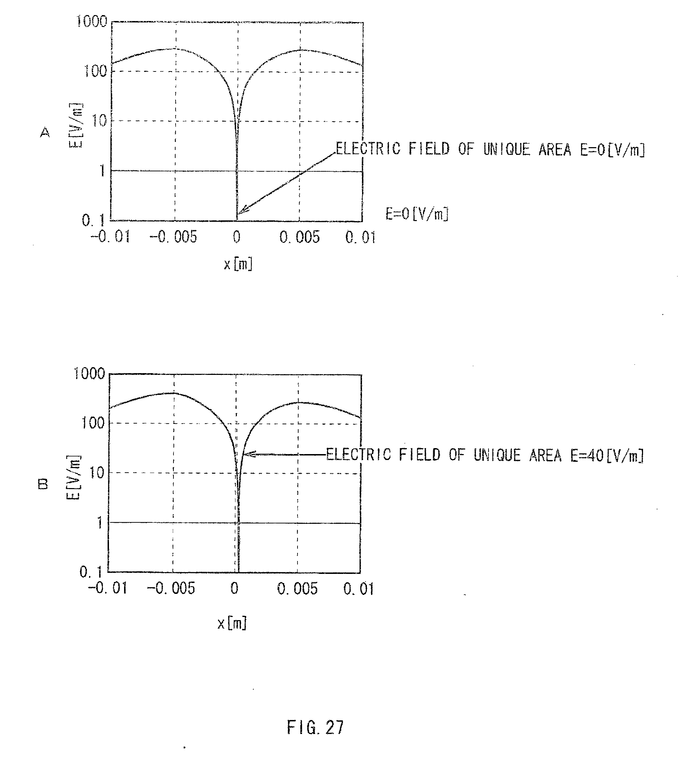

Suppose that the electric charges, +1[C] and -1[C], of the electrodes 11 and 12 have doubled to +2[C] and -2[C]. FIG. 26A shows the results of mapping the electric field strength of the quadrupole before the amounts of charges of the electrodes 11 and 12 change. FIG. 26B shows the results of mapping the electric field strength of the quadrupole after the amounts of charges of the electrodes 11 and 12 have doubled. Moreover, FIG. 27A is a graph showing the electric field strength on the line of y=0 in FIG. 26B. FIG. 27B is a graph showing the electric field strength on the line of y=0 in FIG. 26B. The horizontal axis represents the position of the x-direction.

The following describes the case in which the detection device detects that other electric charges approach the electrodes 11 to 14.

In this case, the electric field strength of the quadrupole and the electric field strength of the electric charges approaching the electrodes 11 to 14 overlap each other. Detection takes place at a position where the electric field becomes 0 as a result of overlapping.

The following describes the frequency of a voltage applied to the electrodes 11 to 14.

In the detection device, a unique area is electrostatically determined by the electric charges of the electrodes 11 to 14. The electrodes 11 to 14 may be charged with electrostatic charges (in the case of a direct current). The sine-wave voltage with a frequency of d<<λ/2π is applied to the electrodes 11 to 14: the wavelength λ of the sine-wave voltage is sufficiently longer than the to-be-detected target 21 or the detection section, i.e. the distance between the electrodes 11 and 12 or between the electrodes 13 and 14, or in other wards the length d of the dipole.

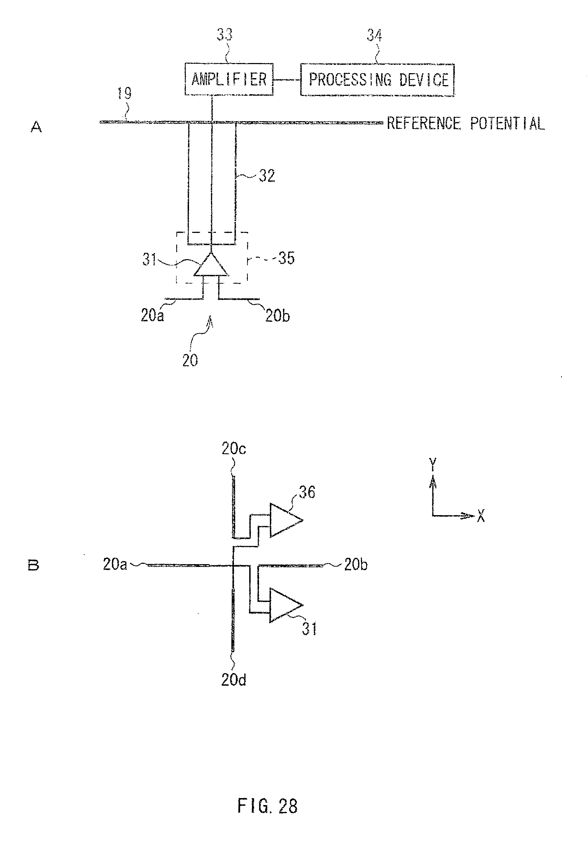

The following describes a specific example of an electric field detection method by the detection electrode 20. FIG. 28A shows an example of the detection electrode 20. In the example here, as shown in FIG. 28A, the detection electrode 20 is made up of an infinitesimal dipole including a pair of electrodes 20a and 20b. The infinitesimal dipole is placed substantially at the same height as the electrodes 11 to 14. The electrodes 20a and 20b that make up the infinitesimal dipole are connected to an input terminal of a differential amplifier 31. In this case, the potential difference between the electrodes 20a and 20b is detected; the detected potential difference is then converted to an electric field. Therefore, it is possible to calculate an electric field positioned at the center of the electrodes 11 to 14. The potential difference between the electrodes 20a and 20b is converted by the differential amplifier 31 with high input impedance into low impedance. Then, signals are transmitted to a processing device 34 through a coaxial cable 32 and an amplifier 33. In this case, the detection section is shielded by a detection section shield 35.

In the case of FIGS. 14A and 14B, in order to detect both electric field vector components Ex and Ey, the detection electrode 20 is for example formed by an infinitesimal dipole including a pair of electrodes 20a and 20b arranged in the direction of the x-axis and an infinitesimal dipole including a pair of electrodes 20c and 20d arranged in the direction of the y-axis, as shown in a two-dimensional diagram of FIG. 28B. The electrodes 20a and 20b that make up the x-axis-direction infinitesimal dipole are connected to the input terminal of the differential amplifier 31. Similarly, the electrodes 20c and 20d that make up the y-axis-direction infinitesimal dipole are connected to an input terminal of a differential amplifier 36. In this case, the potential difference between the electrodes 20a and 20b is detected; the detected potential difference is converted to an electric field. Therefore, it is possible to calculate the electric field vector component Ex. Similarly, the potential difference between the electrodes 20c and 20d is detected; the detected potential difference is converted to an electric field. Therefore, it is possible to calculate the electric field vector component Ey.

As described above, according to the detection device of the first embodiment, to detect the electric field, the detection electrode 20 is provided in the unique area of the quadrupole caused by the electric charges that emerge at the electrodes 11 to 14. Therefore, it is possible to detect the to-be-detected target 21 with high sensitivity and accuracy. The detection device can be used in such detection processes as detecting changes in the positions of the electrodes 11 to 14, detecting changes in the amount of charge of the electrodes 11 to 14, or detecting that other electric charges approach the electrodes 11 to 14.

The following describes a detection device according to a second embodiment of the present invention.

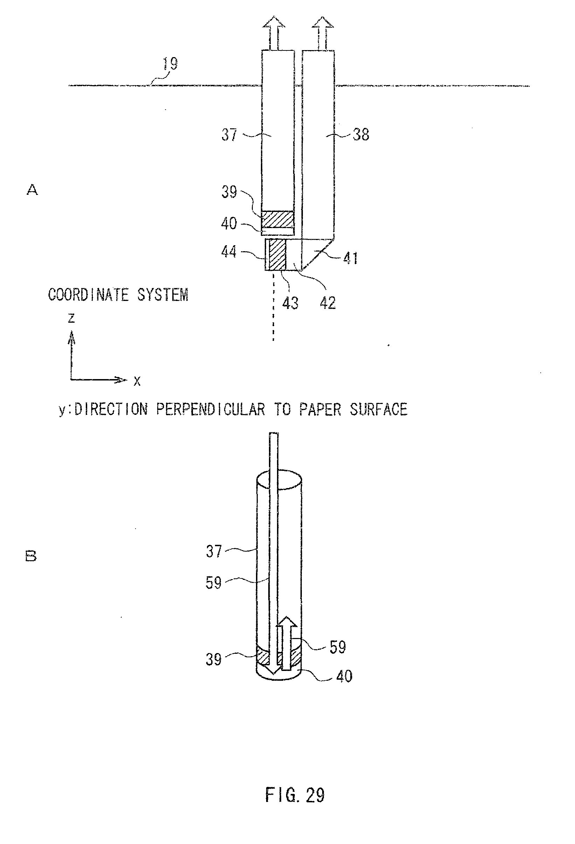

In the detection device, instead of the detection electrode 20 of the detection device of the first embodiment, a detection element that uses electro-optic crystal detects the components Ex and Ey of the electric field vector. FIG. 29A shows one example thereof. As shown in FIG. 29A, optical fibers 37 and 38 pass through holes on the conductor plate 19 in parallel to the z-axis direction. The optical fibers 37 and 38 are used to detect the components Ex and Ey of the electric field vector, respectively. Polarization maintaining fibers are used for the optical fibers 37 and 38. An electro-optic crystal 39 and a mirror 40 are attached to one end of the optical fiber 37. An optical component 41 is attached to one end of the optical fiber 38 to bend light by 90 degrees. The optical component 41 may be a mirror or prism. An optical fiber 42 is attached to the tip of the optical component 41 in parallel to the x-axis direction. An electro-optic crystal 43 and a mirror 44 are attached to the tip of the optical fiber 42. A polarization maintaining fiber is used for the optical fiber 42. In this case, in order to detect the components Ex and Ey of the electric field vector positioned at the same place, the electro-optic crystals 39 and 43 are provided at the same place on the xy-plane so as to be close to each other in the z-axis direction. Lithium niobate (LiNbO3) is for example used for the electro-optic crystals 39 and 43. However, the electro-optic crystals 39 and 43 are not limited to the above. The crystal orientation of the electro-optic crystals 39 and 43 is so determined as to enable a change in the refractive index of the electro-optic crystal 39 to occur only due to the component Ex of the electric field vector as well as to enable a change in the refractive index of the electro-optic crystal 43 to occur only due to the component Ey of the electric field vector. The electro-optic crystals 39 and 43 are so formed as to be sufficiently smaller than the electrodes 11 to 14. In one specific example, the dimensions of the electro-optic crystals 39 and 43 are 270µm×270µm×10µm, which is a square plate. The electro-optic crystals 39 and 43 also could be circular disks with a diameter of 125µm and thickness of 5µm.

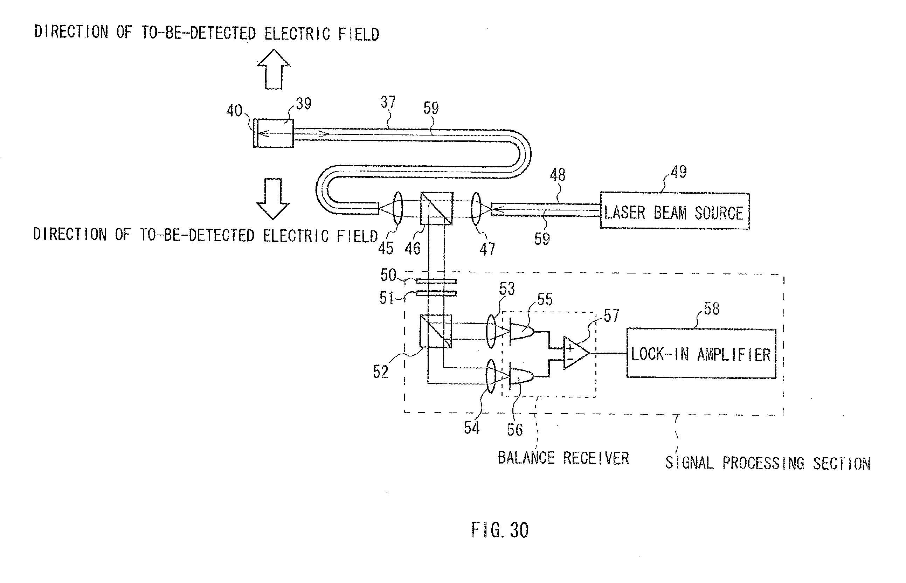

The other ends of the optical fibers 37 and 38 are connected to an optical detection system. FIG. 30 shows one example of the optical detection system. What is described here is the optical detection system connected to the other end of the optical fiber 37. The same is true for the optical detection system connected to the other end of the optical fiber 38. As shown in FIG. 30, the other end of the optical fiber 37 is connected to a laser beam source 49 through a collecting lens 45, a beam splitter 46, a collecting lens 47 and an optical fiber 48. A polarization maintaining fiber is used for the optical fiber 48. On the optical path of one ray emanating from the beam splitter 46, a half-wave plate 50, a quarter-wave plate 51 and a polarization beam splitter 52 are provided. Two rays emanating from the polarization beam splitter 52 are detected by photodiodes 55 and 56 after being collected by collecting lenses 53 and 54. Electric signals from the photodiodes 55 and 56 are input to a differential amplifier 57. The output from the differential amplifier 57 is input to a lock-in amplifier 58.

The following describes a method of detecting the components Ex and Ey of the electric field vector by a detection element that uses the electro-optic crystal.

As shown in FIG. 30, a laser beam 59 having a constant polarization plane from the laser beam source 49 passes through the optical fiber 48, the collecting lens 47, the beam splitter 46, the collecting lens 45, the optical fiber 37 and the electro-optic crystal 39 before entering the mirror 40. After being reflected by the mirror 40, the laser beam 59 passes through the electro-optic crystal 39 again. FIG. 29B shows how the laser beam 59 enters the mirror 40 and gets reflected. When the laser beam 59 passes through the electro-optic crystal 39, the refractive index of the electro-optic crystal 39 changes due to the component Ex of the electric field vector where the electro-optic crystal 39 is positioned. According to the angle that is determined by how much the refractive index has changed, the polarization plane of the laser beam 59 rotates. After passing through the electro-optic crystal 39, the laser beam 59 passes through the optical fiber 37 and the collecting lens 45 before entering the beam splitter 46. One ray emanating from the beam splitter 46 passes through the half-wave plate 50 and the quarter-wave plate 51 before entering the polarization beam splitter 52. Two rays, or polarization components separated by the polarization beam splitter 52 so as to cross each other at right angles, are collected by the collecting lenses 53 and 54 before entering photodiodes 55 and 56 where the two rays are converted into electric signals. The electric signals output from the photodiodes 55 and 56 are input to the differential amplifier 57. In the differential amplifier 57, a difference in the amount of the incident beams of the photodiodes 55 and 56 is detected as a difference of electric signals output from the photodetectors 55 and 56. Based on the electric signals that are thus obtained, the component Ex of the electric field vector can be calculated. Similarly, the component Ey of the electric field vector can be calculated.

If the permittivity of the optical fibers 37, 38, 42 and 48 is different from that of the surrounding area (which is for example an atmosphere), there is a fear that the existence of the optical fibers 37, 38, 42 and 48 could have an effect on the electric field at the detection position. However, the refractive index of the optical fibers 37, 38, 42 and 48 is about 1.1 to 1.5. Accordingly, the relative permittivity εr is approximately equal to 1.2 to 2.5, which is close to the permittivity of the atmosphere (with a relative permittivity of 1). Therefore, the effect of the optical fibers 37, 38, 42 and 48 on the electric field is smaller than the effect of a wire, which is a problem with an electrical detection method, on the electric field.

Except for the above, the second embodiment is the same as the first embodiment.

According to the second embodiment, the same effects as those of the first embodiment can be obtained.

The following describes a vein sensing device according to a third embodiment of the present invention.

The vein sensing device uses a detection device of a quadrupole type of the first embodiment shown in FIG. 12. The vein sensing device detects a pattern of a vein buried underneath a skin.

Since the to-be-detected target 21 is a rod-shaped vein, it is preferable that not only electric field strength but also a to-be-detected electric field vector be taken into consideration. When the vein lies in the x-axis direction, the component Ex of the x-direction electric field vector is not 0 [V/m] . When the vein lies in the y-axis direction, the component Ey of the y-direction electric field vector is not 0[V/m]. That is, it is possible to detect the direction of the vein thanks to the direction of the electric field vector. To detect the electric field, for example, the detection electrode 20 shown in FIG. 28B can be used.

The following proves, with an electromagnetic simulation, that it is possible to detect a vein using a quadrupole electrode for a skin model that is made up of a numerical phantom.

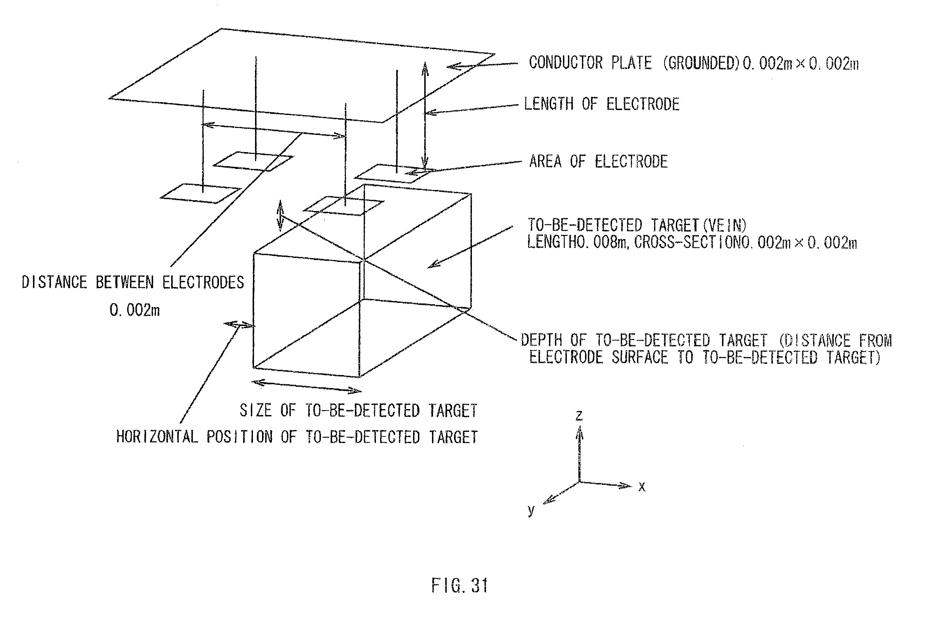

FIG. 31 shows a formulated model. The dimensions of a conductor plate are 0.002m×0.002m. The distance between electrodes is 0.002m. The length of a to-be-detected target is 0.008m. The dimensions of the cross section of a vein are 0.002m×0.002m.

The same sine-wave voltage is applied to two electrodes on one diagonal of a square. The sine-wave voltage whose phase has been shifted by 180 degrees from the above sine-wave voltage is applied to two electrodes on the other diagonal.

The frequency is selected so that as for the electrical characteristics of a living body, the electric conductivity of blood is higher than the electric conductivity of other tissues. According to the simulation, calculation is conducted with a frequency of 1MHz.

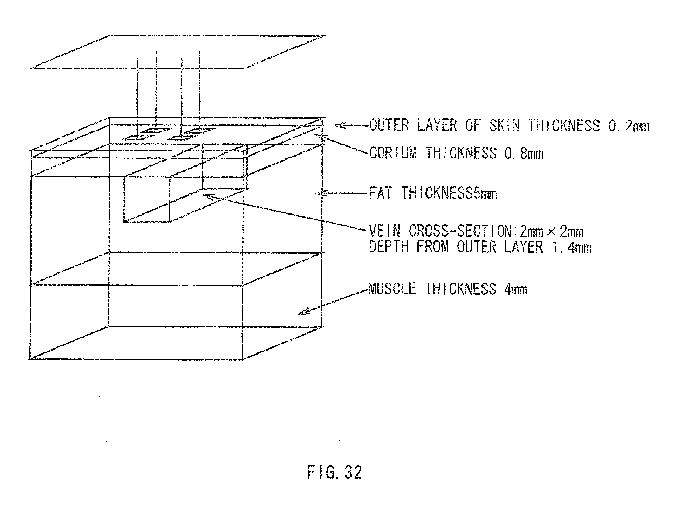

The conditions of the simulation are as follows.
Simulation software: EEM-FDM by Information and Mathematical Science Laboratory Inc.
Calculation method: Maxwell's equations calculated by the finite-difference frequency-domain
Calculation range: x: -4mm to 4mm, y: -3mm to 3mm, z: -5mm to 5mm
Mesh size: 0.0002m
Frequency: 1MHz, Amplitude: 1V
Living-body phantom: a living-body phantom shown in FIG. 32 that represents an outer layer of a human skin. Around the outer layer of a living body tissue, there is a layered structure including, from top to bottom, the outer layer of the skin, corium, fat, and muscle. In such portions as the back of the hand or wrist, a cutaneous vein runs through the fat layer underneath the corium layer. For the electric characteristics of the living body tissue, the values shown in Table 1 by Gabriel are used (See Non-patent Documents 1 to 3)

**(Table 1)**

| | Conductivity [S/m] | Relative permittivity |
|---|---|---|
| Outer layer of skin | 0.01 | 990 |
| Corium | 0.2 | 1832 |
| Fat | 0.04 | 50 |
| Blood | 0.8 | 3026 |
| Muscle | 0.5 | 1836 |

The formulation items are as follows:
a. The horizontal position of a vein - the electric field strength of a unique area
b. The depth of a vein - the electric field strength of a unique area
c. The size of a vein - the electric field strength of a unique area
d. The distance between electrodes - the depth of detection
e. The length of an electrode - the depth of detection
f. The size of a tip of an electrode - the depth of detection

The above will be described one by one.

### a. The horizontal position of a vein - the electric field strength of a unique area

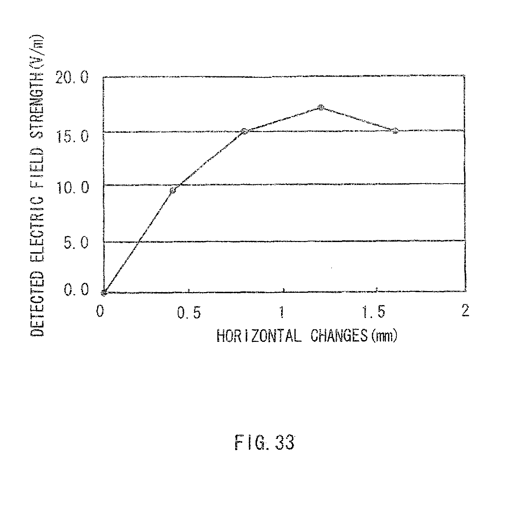

FIG. 33 shows the results of the simulation. It is clear from FIG. 33 that the detected electric field strength is dependent on the relationship between the horizontal position of a to-be-detected target and that of an electrode. When the edge of the vein is positioned at the center of the electrode, the largest electric field strength is detected.

### b. The depth of a vein - the electric field strength of a unique area

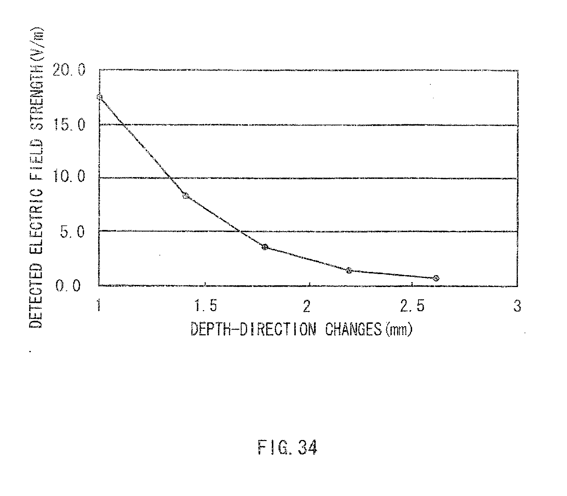

FIG. 34 shows the results of the simulation. It is clear from FIG. 34 that as the vein becomes closer to the outer layer of the skin, the electric field strength rises and can be easily detected.

### c. The size of a vein - the electric field strength of a unique area

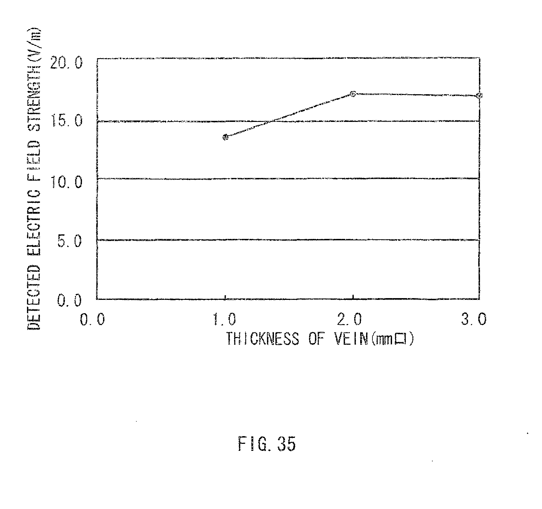

FIG. 35 shows the results of the simulation. It is clear from FIG. 35 that there is little impact of the size of the vein. Since the difference in electric characteristic between the vein and the boundary of the fat is detected, it is considered that even as the vein becomes relatively thick, the detection performance is not affected.

### d. The distance between electrodes - the depth of detection

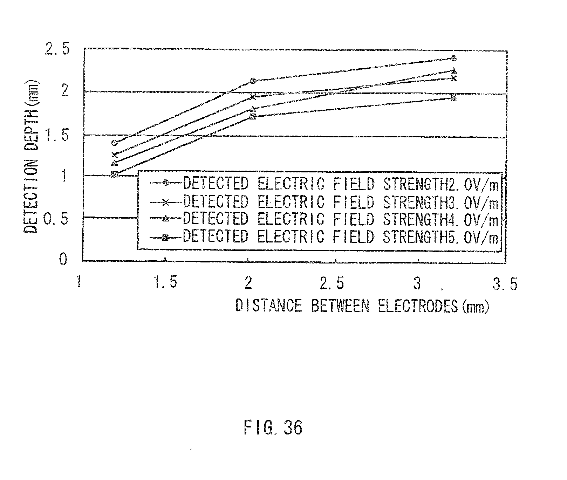

FIG. 36 shows the results of the simulation. It is clear from FIG. 36 that as the distance between electrodes gets larger, it tends to be easier to detect the target positioned at depth.

### e. The length of an electrode - the depth of detection

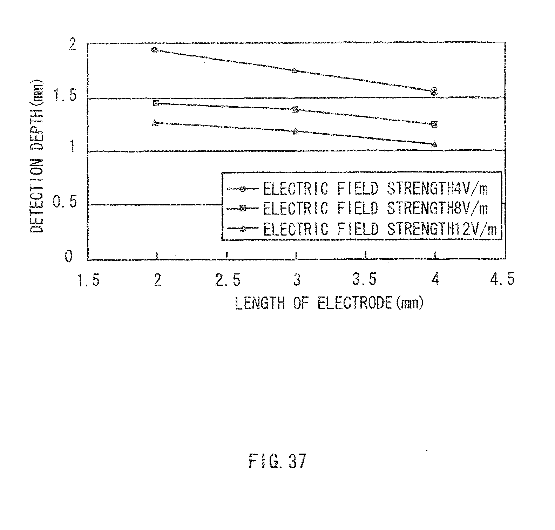

FIG. 37 shows the results of the simulation. It is clear from FIG. 37 that as the electrode becomes shorter, it tends to be easier to detect the target positioned at depth.

The following is a summary of the above. The depth of a vein (blood) around the outer layer of a skin inside a living body is dependent on the horizontal position and depth of the vein. In the horizontal direction, the sensitivity to positional changes is significantly high; the amount of detection is smaller for a target positioned at depth. It is found that it is possible to dramatically improve the amount of detection with the use of a method of increasing the area of the plate of the electrode tip portion or a method of narrowing the distance between electrodes.

According to the vein sensing device of the third embodiment, it is possible to non-invasively detect a vein inside a living body with high sensitivity and accuracy from above the outer layer of the skin. It is possible to accurately detect the pattern of the vein. For example, the vein sensing device can be applied to the case where the vein sensing device is put on an arm vein to detect the pattern of the vein for the purpose of vein personal authentication.

As a conventional method to detect a vein pattern inside a living body from above the outer layer of the skin, there is an impedance measurement method. However, the following are the problems with the impedance measurement method: varying and unstable impedance depending on how the electrode is put on the skin; perspiration that affects the impedance measurement method; unclear differences between a vein and any parts other than the vein; the electromotive force caused by two kinds of electrodes (metal) put on the skin as the electrodes come in contact with, which gives rise to noise during the impedance measurement process. On the other hand, the vein sensing device of the third embodiment does not have such problems. Table 2 shows a comparison of the detection method of the present method and the detection method of the impedance measurement method.

**(Table 2)**

| Item | Present method | Impedance method |
|---|---|---|
| Detection physical quantity | Boundary between two tissues of living body that are different in conductivity | Impedance of tissues of living body |
| To-be-detected target | Case where there is boundary between two tissues of living body beneath quadrupole (Planar resolution is high because boundary is beneath quadrupole) | Tissues around impedance measurement electrode (There is dominance beneath impedance measurement electrode. However, planar resolution cannot be raised because of effects of impedance of portion at distance. |
| Accuracy | Clearly detect vein and other parts | Unclear differences between vein and other parts (Because of current flowing through broad area of living body) |
| Detection performance | Possible to detect depth of about 2mm | Difficult to detect vein at depth |
| Effects of electrodes that come in contact with skin | Possible to detect in non-contact manner | Varying contact resistance resulting from unstable contact and electromotive force caused by contact give rise to noise |

The following describes a vein sensing device according to a fourth embodiment of the present invention.

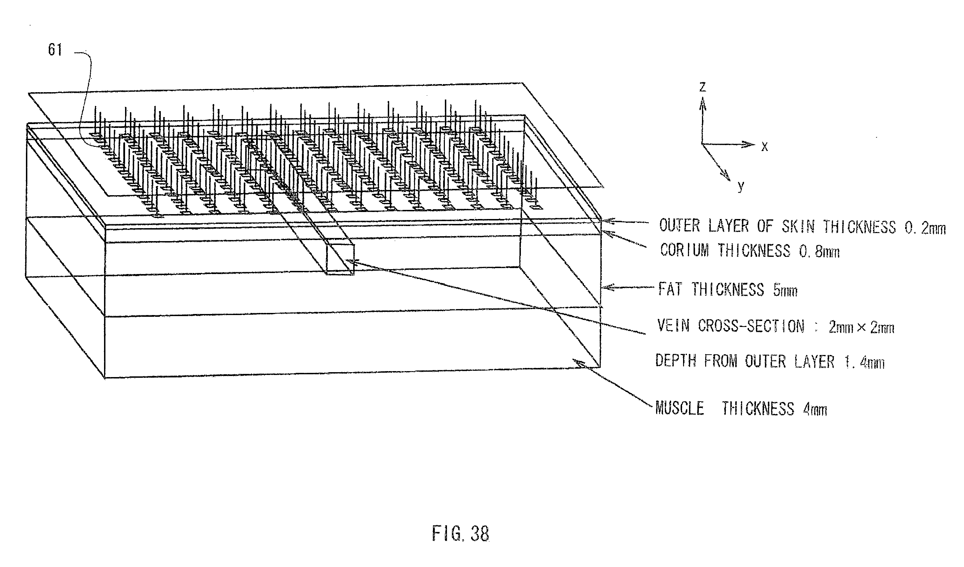

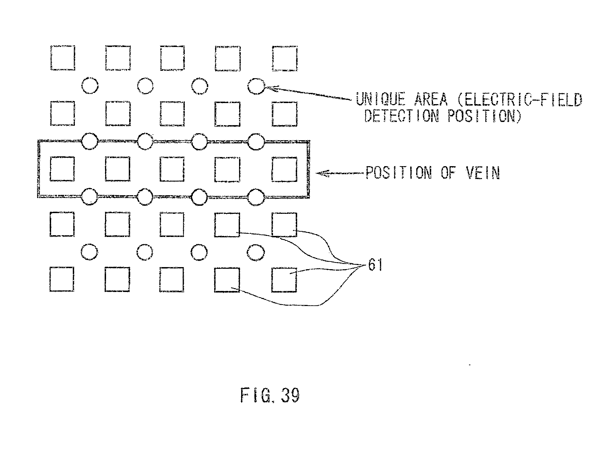

As shown in FIG. 38, in the vein sensing device, a matrix array electrode where many electrodes 61 are arranged in the form of matrix array within the xy-plane is used. The matrix array electrode is equivalent to the one where many basic units, one of which is a quadrupole-type electrode used in the detection device of the first embodiment, are arranged in the form of two-dimensional array. FIG. 39 is an enlarged view of a portion of the matrix array electrode. In FIG. 39, four electrodes 61 on the vertexes of a square indicated by dashed line are equivalent to the electrodes 11 to 14 of the detection device of the first embodiment and serve as one basic unit. A unique area (electric-field detection position) is at the center of the one basic unit.

With the use of the matrix array electrode shown in FIGS. 38 and 39 and the same numerical phantom as that of the third embodiment, an electromagnetic field simulation is conducted.

The conditions of the simulation are as follows.
Simulation software: EEM-FDM by Information and Mathematical Science Laboratory Inc.
Calculation method: Maxwell's equations calculated by the finite-difference frequency-domain
Calculation range: x: -0.003 to 0.031m, y: -0.003 to 0.031m, z: -0.05 to 0.05m
Mesh size: 0.002m
Frequency: 1MHz, Amplitude: 1V
Living-body phantom: a living-body phantom shown in FIG. 32 that represents an outer layer of a human skin.

The electrical characteristics are shown in Table 1.

The number of electrodes 61 arranged comes to 14×14=196.

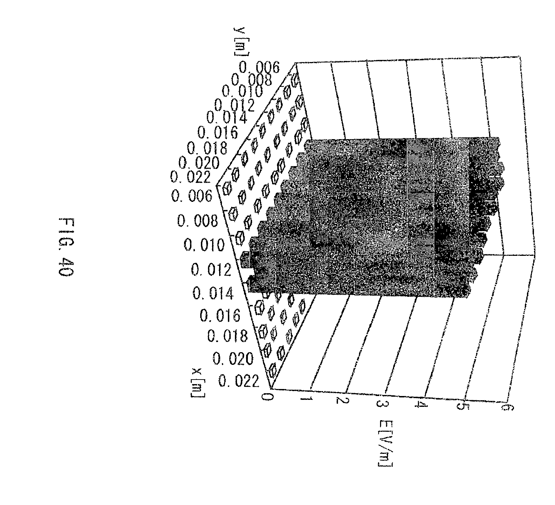

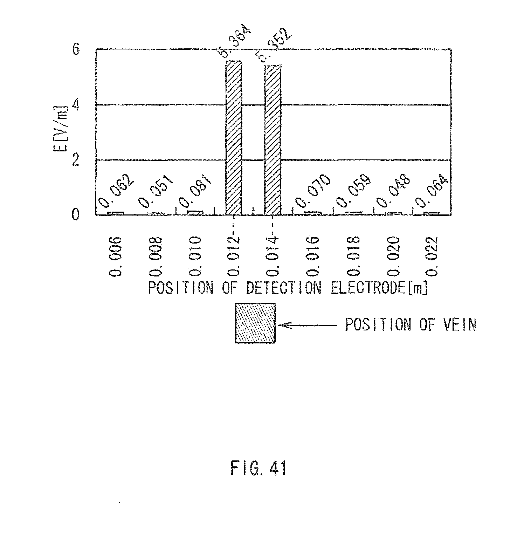

FIG. 40 shows the electric field strength of 9×9 detection positions at a central portion out of 13×13 detection positions at the center of four electrodes 61 that make up one basic unit. FIG. 41 shows the cross-section of y=0.014 shown in FIG. 40. The electric field strength of a portion corresponding to the edge of a vein is about 5.3 [V/m]. The electric field strength of the remaining unique area is less than or equal to 0.08 [V/m] . Thanks to the difference in the electric field strength, it is possible to detect the position of a vein.

The use of the matrix array electrode helps to improve both the horizontal resolution and the depth-direction detection performance. The reasons will be described below.

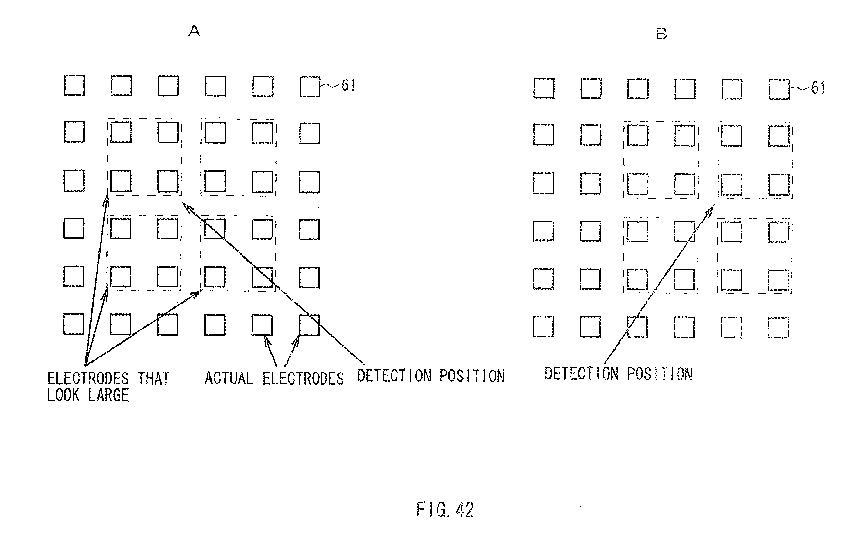

As described above, as for the detection performance of the quadrupole-type electrode, it is possible to detect a deeper vein as the area of the electrode is increased. However, as the area of the electrode increases, the planar resolution decreases. Accordingly, a plurality of electrodes in the matrix array electrode are regarded as a group to which the same electric signals (voltage) are applied. In this manner, the electrodes are apparently regarded as one large electrode. In this case, an atomic unit, which is one basic unit, and a group of the basic units are the same in configuration. Therefore, the configuration is referred to as a fractal structure. FIG. 42A shows a portion of the matrix array electrode. In the case of FIG. 42A, the same electric signals are applied to four electrodes 61 encircled by dashed lines. Therefore, the electrodes 61 are handled as one electrode whose area is apparently large in size. In this case, four electrodes whose area is apparently large in size are used to form a quadrupole. Therefore, it is possible to detect a to-be-detected target (vein) positioned deeper. FIG. 42B shows an example in which a large electrode is made up of different electrodes 61 from those of FIG. 42A.

When the matrix array electrode having such a fractal structure is used for detection, scanning is for example performed in the following manner.

Scanning of the matrix array electrode is performed several times. In the first scanning process, the four electrodes 61 encircled by dotted lines in FIG. 42A are used for detection. In the second scanning process, the four electrodes 61 encircled by dotted lines in FIG. 42B are used for detection. In this case, the configuration of the electrodes is as follows: the electrodes have moved in the x-direction compared with those in FIG. 42A. In the third scanning process, the following configuration of the electrodes is used for detection: the electrodes have moved in the y-direction from where the electrodes are as shown FIG. 42A. In the fourth scanning process, the following configuration of the electrodes is used for detection: the electrodes have moved in the y-direction from where the electrodes are as shown FIG. 42B.

As described above, according to the vein sensing device of the fourth embodiment, the large electrode is formed by the electrical switching of the matrix array electrode. The planar-direction resolution is designed to be dependant on the distance between the electrodes 61 of one basic unit. It is possible to scan with the above resolution. Therefore, in the vein sensing device, it is possible to obtain the depth-direction detection performance that matches that of the physically large electrode. Since scanning is possible on a per-one-basic-unit-electrode basis, it is possible to obtain a high horizontal resolution, which cannot be obtained by a simply large electrode.

Incidentally, in the example shown in FIGS. 42A and 42B, 2×2 electrodes 61 are combined into a large electrode. However, in general, n×n electrodes may be combined into a large electrode. Therefore, it is also possible to scan not only in the planar direction but also in the depth direction. Thus, three-dimensional scanning is possible.

The following describes a scanning probe microscope according to a fifth embodiment of the present invention.

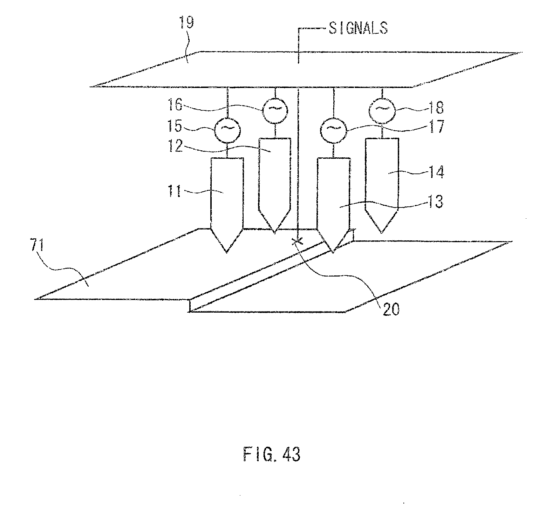

As shown in FIG. 43, in the scanning probe microscope, four electrodes 11 to 14 are formed in a sharp-pointed shape like a probe of a conventional scanning probe microscope. Many basic units, each of which consists of the electrodes 11 to 14, are arranged in the form of two-dimensional array to form a matrix array electrode. Sine-wave voltages that are the same in polarity and phase are applied across the electrodes 11 and 14. Sine-wave voltages whose phase is shifted by 180 degrees from the above sine-wave voltages are applied across the electrodes 12 and 13. A detection electrode 20 is provided in a unique area at the center of the electrodes 11 to 14.

The operation of the scanning probe microscope will be described. As shown in FIG. 43, a sample 71 is put on a stage (not shown) . The sharp-pointed tips of electrodes 11 to 14, many of which are arranged in the form of two-dimensional array, are brought closer to the surface of the sample 71. The stage remains fixed and electric scanning of the matrix array electrode, which consists of two-dimensional arrays of electrodes 11 to 14, is performed. At this time, the electric field of the unique area at the center of the electrodes 11 to 14 changes with the uneven surface of the sample 71. Therefore, it is possible to measure the unevenness of the surface of the sample 71 by detecting the changes.

According to the fifth embodiment, it is possible to realize the scanning probe microscope based on a novel principle. Unlike a conventional atom force microscope, a structure for moving a sample in the horizontal direction is not required in the scanning probe microscope. It is unnecessary to use a cantilever that mechanically operates. Since the scanning probe microscope works in a non-contact manner, the sample is not damaged and there are no other effects on the sample. It is possible to accurately scan the uneven surface of the sample at high speed.

The following describes a distortion detection device according to a sixth embodiment of the present invention.

The distortion detection device detects the distortion of a structure such as a building and of land by detecting a change in the position of a multipole electrode through a change in the electric field strength of a unique area.

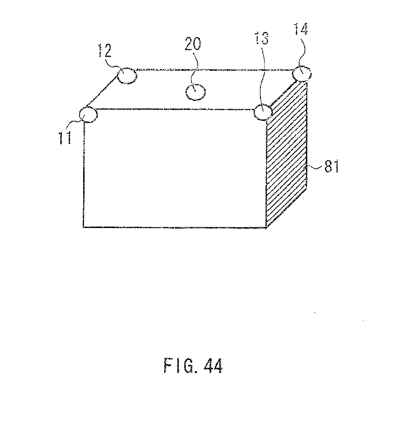

FIG. 44 shows one example. As shown in FIG. 44, a quadrupole-type electrode, or electrodes 11 to 14, is for example placed on the four corners of the top surface of a building 81. For example, if the building 81 is one room, the quadrupole-type electrode may be placed on the four corners of the ceiling. A detection electrode 20 is provided in an unique area at the center of the electrodes 11 to 14.

In the distortion detection device, if the building 81 is not distorted, the electric field of the unique area is 0 [V/m]. However, if the positions of the electrodes 11 to 14 change as the building 81 is distorted, the electric field detected by the detection electrode 20 does not satisfy 0 [V/m]. Therefore, it is possible to predict the distortion of the building 81 thanks to the change of the electric field.

According to the sixth embodiment, it is possible to realize the distortion detection device based on a novel principle.

The following describes a metal detection device according to a seventh embodiment of the present invention.

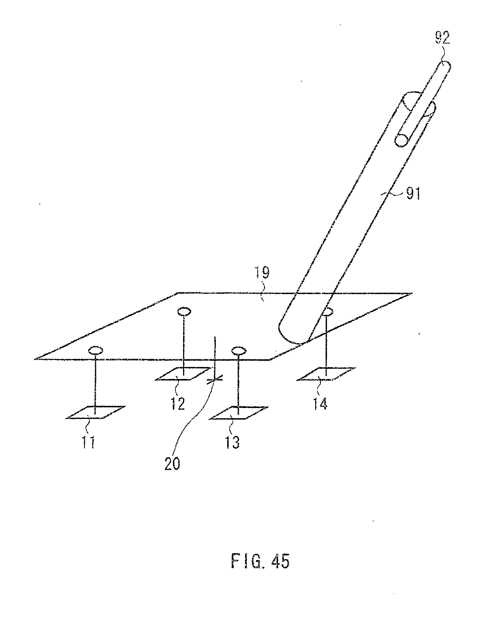

As shown in FIG. 45, in the metal detection device, a supporting rod 91 is attached to the surface of a conductor plate 19 of a detection device of a quadrupole type that is the same as in the first embodiment. A handle 92 is attached to one end of the supporting rod 91.

When an object whose conductivity is different or an object whose permittivity is different comes closer to the detection electrode 20, the metal detection device detects the object. Specifically, the to-be-detected object 21 is, for example, a buried metal object around the surface of the earth, an object that is different in electric characteristic from the ground, a metal obj ect embedded in a wall, or the like.

The operation of the metal detection device will be described.

An operator uses a hand to grab the handle 92 and moves the metal detection device along the surface of the ground in the horizontal direction. At this time, above an uniform object, electric charges that occur on the electrodes 11 to 14, i.e. the electric fields caused by the quadrupole, are balanced relative to the electrodes 11 to 14. The electric field strength is 0 [V/m] at the center of the electrodes 11 to 14. If a metal object or an object whose electric characteristic is different exists away from the center of the electrodes 11 to 14, the balance of the electric fields caused by the electric charges of the electrodes 11 to 14 is lost. The detection electrode 20 positioned at the center detects an electric field other than the one that is 0 [V/m]. The change of the electric field enables metal to be detected.

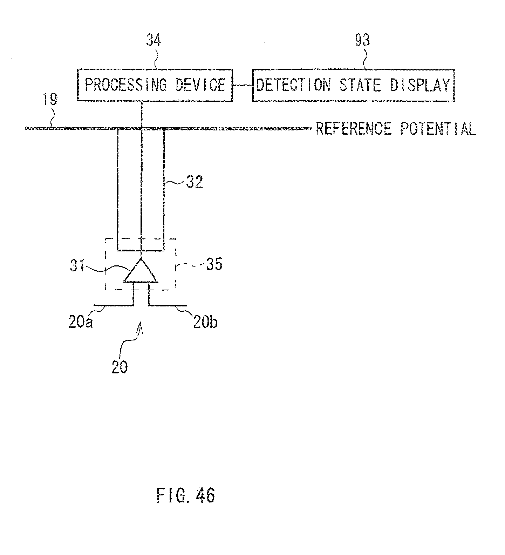

The electric-field detection of the detection electrode 20 can be carried out with the use of the same detection system, which is shown in FIG. 46, as that shown in FIGS. 28A and 28B. In this case, the signals conveyed to a processing device 34 are transmitted to a display device 93 where a detection state can be displayed.

According to a seventh embodiment, it is possible to realize the metal detection device based on a novel principle.

The following describes a scanning probe microscope according to an eighth embodiment of the present invention.

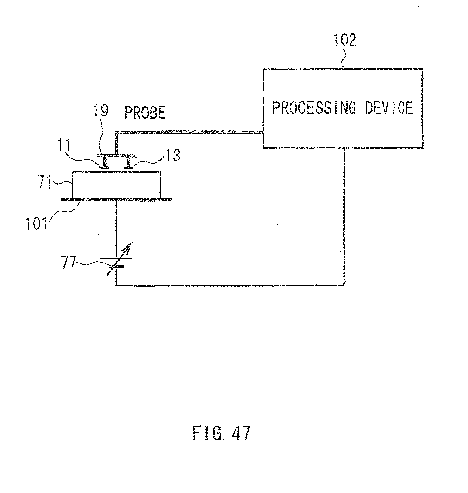

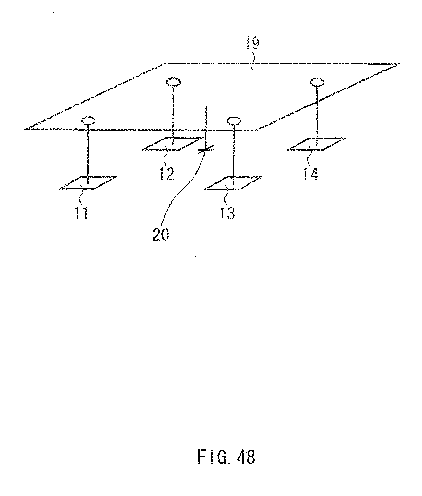

As shown in FIG. 47, in the scanning probe microscope, the detection device of the first embodiment, which serves as a probe, is brought closer to the surface of a sample 71 put on a stage 101. Then, the stage 101 moves within a horizontal plane. In this case, by detecting the changes of the electric field of a unique area at the center of the four electrodes 11 to 14 of the detection device resulting from the unevenness of the surface of the sample 71, it is possible to detect the unevenness of the surface of the sample 71. An output-signal process of the detection device is performed by a processing device 102. The movement of the stage 101 is done by a driving power source 103. FIG. 48 shows one example of the configuration of the detection device that is used as a probe.

According to the scanning probe microscope, since the detection device does not touch the surface of the sample, the sample is not damaged. Moreover, since the scanning probe microscope does not use a cantilever that mechanically operates like a conventional atom force microscope, it is possible to accurately scan the uneven surface of the sample at high speed.

According to the eighth embodiment, it is possible to realize the scanning probe microscope based on a novel principle.

The above has provided a detailed description of the embodiments of the present invention. However, the present invention is not limited to the above embodiments. Various modifications may be made based on the technical ideas of the present invention.

For example, the numeric values, structures, arrangements, shapes and the like described in the above embodiments are just examples. Therefore, different numeric values, structures, arrangements, shapes and the like may be used when necessary.

## Claims

1. A detection device **characterized by** comprising:
m electrodes (11, 12, 13, 14) that generate at least around one straight line m rotationally symmetric electric charges, where m is an even number that is greater than or equal to 4, whose total amount of charge is substantially 0; and
at least one electric field detection element (20) that detects an electric field on the straight line.

2. The detection device according to claim 1, **characterized in that**
the signs of adjacent electric charges of the m electric charges are different.

3. The detection device according to claim 1, **characterized in that**
the absolute values of the m electric charges are equal to each other.

4. The detection device according to claim 1, **characterized in that**
an electric field detection element and a wire (32) thereof are disposed on the straight line.

5. The detection device according to claim 1, **characterized in that**
the m electric charges make up a multipole;
the m electric charges make up a planar 2n-pole, where n is an integer that is greater than or equal to 2; or
the m electric charges emerge at a vertex of a regular polyhedron or quasi-regular polyhedron.

6. The detection device according to claim 1, **characterized in that**
a change of the position of at least one electrode among the m electrodes is detected; or
a change of the amount of charge of electric charges that emerge on at least one electrode among the m electrodes is detected.

7. The detection device according to claim 1, **characterized in that**
an electric charge or object (21; 71) outside the m electrodes is detected.

8. The detection device according to claim 1, **characterized in that**
the m electric charges are generated by applying an alternating voltage to the m electrodes.

9. The detection device according to claim 1, **characterized in that**
when the m electric charges are generated by applying a sine-wave alternating voltage to the m electrodes, d<<λ/2π where λ represents the length of the sine wave and d represents the length of a dipole (20a, 20b; 20c, 20d) that makes up the m electric charges.

10. The detection device according to claim 1, **characterized in that**
the m electric charges are static electric charges.

11. The detection device according to claim 1, **characterized in that**
the m electrodes are point electrodes or planar electrodes.

12. The detection device according to claim 1, **characterized in that**
with the m electrodes (11, 12, 13, 14; 61) as one basic unit, a plurality of basic units are arranged in one-dimensional or two-dimensional array.

13. A detection device that detects a to-be-detected target (21) or a change of the state of the to-be-detected target (21), **characterized by** comprising:
a plurality of electric field applying means (11, 12, 13, 14) for applying electric fields to the to-be-detected target;
electric field detection means (20) for detecting an electric field of a detection area to which the to-be-detected target can be brought adjacent; and
processing means (34; 102) for detecting a change of the electric field of the detection area detected by the electric field detection means and detecting the to-be-detected target or a change of the state of the to-be-detected target, wherein
when the to-be-detected target is not adjacent to the detection area or is in a predetermined state, a plurality of the electric field applying means applies electric fields in such a way that the electric fields applied from a plurality of the electric field applying means diminish each other and an electric field adjacent to the detection area and the electric field detection means comes to substantially 0.

14. A detection method **characterized in that**
m rotationally symmetric electric charges (11, 12, 13, 14), where m is an even number that is greater than or equal to 4, whose total amount of charge is substantially 0 are generated at least around one straight line, and an electric field on the straight line is detected.

15. The detection device according to Claim 1, **characterized in that** the detection device is a vein sensing device, part of a scanning probe microscope, a distortion detection device or a metal detection device.

## Patentansprüche

1. Detektionsvorrichtung, **dadurch gekennzeichnet, dass** sie Folgendes umfasst:
m Elektroden (11, 12, 13, 14), die mindestens um eine gerade Linie m rotationssymmetrische elektrische Ladungen erzeugen, wobei m eine gerade Zahl ist, die größer oder gleich 4 ist, und deren gesamte Ladungsmenge im Wesentlichen 0 ist; und
mindestens ein Detektionselement (20) für ein elektrisches Feld, das ein elektrisches Feld auf der geraden Linie detektiert.

2. Detektionsvorrichtung nach Anspruch 1, **dadurch gekennzeichnet, dass**
die Vorzeichen benachbarter elektrischer Ladungen der m elektrischen Ladungen verschieden sind.

3. Detektionsvorrichtung nach Anspruch 1, **dadurch gekennzeichnet, dass**
die absoluten Werte der m elektrischen Ladungen einander gleich sind.

4. Detektionsvorrichtung nach Anspruch 1, **dadurch gekennzeichnet, dass**
ein Detektionselement für ein elektrisches Feld und ein Draht (32) davon auf der geraden Linie angeordnet sind.

5. Detektionsvorrichtung nach Anspruch 1, **dadurch gekennzeichnet, dass**
die m elektrischen Ladungen einen Multipol bilden;
die m elektrischen Ladungen einen ebenen 2n-Pol bilden, wobei n eine ganze Zahl ist, die größer oder gleich 2 ist; oder
die m elektrischen Ladungen an einer Ecke eines regelmäßigen Polyeders oder eines quasi-regelmäßigen Polyeders auftreten.

6. Detektionsvorrichtung nach Anspruch 1, **dadurch gekennzeichnet, dass**
eine Veränderung der Position von mindestens einer Elektrode unter den m Elektroden detektiert wird; oder
eine Veränderung der Ladungsmenge von elektrischen Ladungen, die an der mindestens einen Elektrode unter den m Elektroden auftreten, detektiert wird.

7. Detektionsvorrichtung nach Anspruch 1, **dadurch gekennzeichnet, dass**
eine elektrische Ladung oder ein elektrisches Objekt (21; 71) außerhalb der m Elektroden detektiert wird.

8. Detektionsvorrichtung nach Anspruch 1, **dadurch gekennzeichnet, dass**
die m elektrischen Ladungen durch Anlegen einer Wechselspannung an die m Elektroden erzeugt werden.

9. Detektionsvorrichtung nach Anspruch 1, **dadurch gekennzeichnet, dass**
dann, wenn die m elektrischen Ladungen durch Anlegen einer sinusförmigen Wechselspannung an die m Elektroden erzeugt werden, d « λ/2π gelten soll, wobei λ die Länge der Sinuswelle darstellt und d die Länge eines Dipols (20a, 20b; 20c, 20d), der die m elektrischen Ladungen bildet, darstellt.

10. Detektionsvorrichtung nach Anspruch 1, **dadurch gekennzeichnet, dass**
die m elektrischen Ladungen statische elektrische Ladungen sind.

11. Detektionsvorrichtung nach Anspruch 1, **dadurch gekennzeichnet, dass**
die m Elektroden Punktelektroden oder ebene Elektroden sind.

12. Detektionsvorrichtung nach Anspruch 1, **dadurch gekennzeichnet, dass**
mit den m Elektroden (11, 12, 13, 14; 61) als eine Basiseinheit mehrere Basiseinheiten in einer eindimensionalen oder zweidimensionalen Anordnung angeordnet sind.

13. Detektionsvorrichtung, die ein Ziel (21), das detektiert werden soll, oder eine Veränderung des Zustands des zu detektierenden Ziels (21) detektiert, **dadurch gekennzeichnet, dass** sie Folgendes umfasst:
mehrere Mittel (11, 12, 13, 14), um ein elektrisches Feld anzulegen, um an das zu detektierende Ziel elektrische Felder anzulegen;
Mittel (20) zur Detektion eines elektrischen Felds, um ein elektrisches Feld einer Detektionsfläche, in deren Nachbarschaft das zu detektierende Ziel gebracht werden kann, zu detektieren; und
Verarbeitungsmittel (34; 102) zum Detektieren einer Veränderung des elektrischen Felds der Detektionsfläche, die durch die Mittel zur Detektion des elektrischen Felds detektiert worden ist, und zum Detektieren des zu detektierenden Ziels oder einer Veränderung des Zustands des zu detektierenden Ziels, wobei
dann, wenn das zu detektierende Ziel nicht zu der Detektionsfläche benachbart ist oder sich nicht in einem vorgegebenen Zustand befindet, mehrere der Mittel, die ein elektrisches Feld anlegen, elektrische Felder derart anlegen, dass sich die elektrischen Felder, die von mehreren der Mittel zur Anlegung eines elektrischen Felds angelegt worden sind, gegenseitig verringern und dass ein elektrisches Feld, das zu der Detektionsfläche und zu den Mitteln zur Detektion des elektrischen Felds benachbart ist, im Wesentlichen den Wert 0 ergibt.

14. Detektionsverfahren, **dadurch gekennzeichnet, dass** m rotationssymmetrische elektrische Ladungen (11, 12, 13, 14), wobei m eine gerade Zahl ist, die größer oder gleich 4 ist, und deren gesamte Ladungsmenge im Wesentlichen 0 ist, mindestens um eine gerade Linie erzeugt werden, und dass ein elektrisches Feld auf der geraden Linie detektiert wird.

15. Detektionsvorrichtung nach Anspruch 1, **dadurch gekennzeichnet, dass** die Detektionsvorrichtung eine Erfassungsvorrichtung für eine Vene, Teil eines Rastersondenmikroskops, einer Detektionsvorrichtung für eine Verzerrung oder einer Detektionsvorrichtung für Metall ist.

## Revendications

1. Dispositif de détection **caractérisé en ce qu'**il comprend :
m électrodes (11, 12, 13, 14) qui génèrent au moins autour d'une ligne droite m charges électriques symétriques en rotation, où m est un nombre pair qui est supérieur ou égal à 4, dont la quantité totale de charge vaut sensiblement 0 ; et
au moins un élément de détection de champ électrique (20) qui détecte un champ électrique sur la ligne droite.

2. Dispositif de détection selon la revendication 1, **caractérisé en ce que**
les signes de charges électriques adjacentes parmi les m charges électriques sont différents.

3. Dispositif de détection selon la revendication 1, **caractérisé en ce que**
les valeurs absolues des m charges électriques sont égales les unes aux autres.

4. Dispositif de détection selon la revendication 1, **caractérisé en ce que**
un élément de détection de champ électrique et un fil (32) de celui-ci sont disposés sur la ligne droite.

5. Dispositif de détection selon la revendication 1, **caractérisé en ce que**
les m charges électriques constituent un multipôle ;
les m charges électriques constituent un 2n-pôle plan, où n est un entier qui est supérieur ou égal à 2 ; ou
les m charges électriques apparaissent à un sommet d'un polyèdre régulier ou d'un polyèdre quasiment régulier.

6. Dispositif de détection selon la revendication 1, **caractérisé en ce que**
un changement de la position d'au moins une électrode parmi les m électrodes est détecté ; ou
un changement de la quantité de charge des charges électriques qui apparaissent sur au moins une électrode parmi les m électrodes est détecté.

7. Dispositif de détection selon la revendication 1, **caractérisé en ce que**
une charge électrique ou un objet (21 ; 71) à l'extérieur des m électrodes est détecté.

8. Dispositif de détection selon la revendication 1, **caractérisé en ce que**
les m charges électriques sont générées en appliquant une tension alternative aux m électrodes.

9. Dispositif de détection selon la revendication 1, **caractérisé en ce que**
quand les m charges électriques sont générées en appliquant une tension alternative sinusoïdale aux m électrodes, d « λ/2Π où λ représente la longueur de l'onde sinusoïdale et d représente la longueur d'un dipôle (20a, 20b ; 20c, 20d) qui compose les m charges électriques.

10. Dispositif de détection selon la revendication 1, **caractérisé en ce que**
les m charges électriques sont des charges électriques statiques.

11. Dispositif de détection selon la revendication 1, **caractérisé en ce que**
les m électrodes sont des électrodes ponctuelles ou des électrodes planes.

12. Dispositif de détection selon la revendication 1, **caractérisé en ce que**
avec les m électrodes (11, 12, 13, 14 ; 61) comme unité de base, une pluralité d'unités de base sont agencées en un réseau unidimensionnel ou bidimensionnel.

13. Dispositif de détection qui détecte une cible à détecter (21) ou un changement de l'état de la cible à détecter (21), **caractérisé en ce qu'**il comprend :
une pluralité de moyens d'application de champ électrique (11, 12, 13, 14) pour appliquer des champs électriques à la cible à détecter ;
un moyen de détection de champ électrique (20) pour détecter un champ électrique d'une zone de détection en contiguïté de laquelle la cible à détecter peut être placée ; et
un moyen de traitement (34 ; 102) pour détecter un changement du champ électrique de la zone de détection détecté par le moyen de détection de champ électrique et détecter la cible à détecter ou un changement de l'état de la cible à détecter, dans lequel
quand la cible à détecter n'est pas contiguë à la zone de détection ou est dans un état prédéterminé, une pluralité des moyens d'application de champ électrique applique des champs électriques de telle sorte que les champs électriques appliqués depuis une pluralité des moyens d'application de champ électrique s'atténuent les uns les autres et un champ électrique contigu à la zone de détection et au moyen de détection de champ électrique atteigne sensiblement 0.

14. Procédé de détection **caractérisé en ce que** m charges électriques symétriques en rotation (11, 12, 13, 14), où m est un nombre pair qui est supérieur ou égal à 4, dont la quantité totale de charge vaut sensiblement 0 sont générées au moins autour d'une ligne droite, et un champ électrique sur la ligne droite est détecté.

15. Dispositif de détection selon la revendication 1, **caractérisé en ce que** le dispositif de détection est un dispositif de détection de veines, une partie d'un microscope en champ proche, un dispositif de détection de déformations ou un dispositif de détection de métaux.
